# EUROPEAN PATENT SPECIFICATION

(11) **EP 1 628 990 B1**
(45) Date of publication and mention of the grant of the patent: **02.12.2009**
(21) Application number: 04732113.8
(22) Date of filing: 11.05.2004
(51) Int. Cl.: C07H 17/08, A61K 31/7048

(54) **NOVEL 14 AND 15 MEMBERED RING COMPOUNDS**
NEUE 14- UND 15-GLIEDRIGE RINGVERBINDUNGEN
NOUVEAUX COMPOSES CYCLIQUES DE 14 ET DE 15 CHAINONS

(30) Priority: 13.05.2003 GB 0310980
(43) Date of publication of application: 01.03.2006
(73) Proprietor: GLAXO GROUP LIMITED, Greenford, Middlesex UB6 ONN (GB); Pliva - Istrazivacki Institut d.o.o., 10000 Zagreb (HR)
(72) Inventor: ALIHODZIC, Sulejman, Pliva-Istrazivacki Ins. d.o.o, 10000 Zagreb (HR); JARVEST, Richard Lewis, GlaxoSmithKline, Stevenage, Hertfordshire SG1 2NY (GB); PALEJ, Ivana, Pliva-Istrazivacki Ins. d.o.o., 10000 Zagreb (HR)
(74) Representative: Hambleton, Bernadette Angelina
(86) International application number: PCT/EP2004/005084
(87) International publication number: WO 2004/101588

(56) References cited:
- EP-A- 0 895 999
- WO-A-03/042228
- WO-A-2004/039822
- US-B1- 6 300 316

## Description

The present invention relates to novel semi-synthetic macrolides having antimicrobial activity, in particular antibacterial activity. More particularly, the invention relates to 14- and 15-membered macrolides substituted at the 4" position, to processes for their preparation, to compositions containing them and to their use in medicine.

Macrolide antibacterial agents are known to be useful in the treatment or prevention of bacterial infections. However, the emergence of macrolide-resistant bacterial strains has resulted in the need to develop new macrolide compounds. For example, EP 0 895 999 or US 6 300 316 describe derivatives modified at the 4" position of the macrolide ring having antibacterial activity.

According to the present invention, we have now found novel 14- and 15-membered macrolides substituted at the 4" position which also have antimicrobial activity.

Thus, the present invention provides compounds of general formula (I) 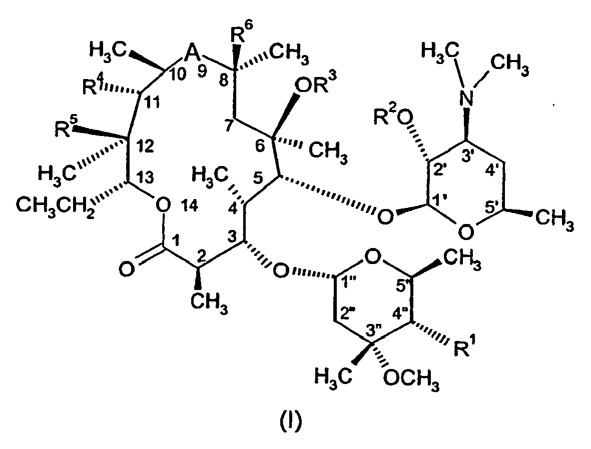 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)N(R¹¹)(CH₂)_{d}XR¹²;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹³,
R⁵ is hydroxy, or
R⁴ and R⁵ taken together with the intervening atoms form a cyclic group having the following structure: 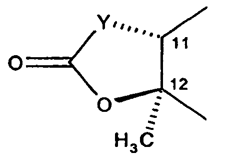 wherein Y is a bivalent radical selected from -CH₂-, -CH(CN)-, -O-, -N(R¹⁴)- and-CH(SR¹⁴)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁵R¹⁶ or-C(O)R¹⁵, or
R⁸ and R⁹ together form =CH(CR¹⁵R¹⁶)_{f}aryl, =CH(CR¹⁵R¹⁶)_{f}heterocyclyl, =CR¹⁵R¹⁶ or =C(R¹⁵)C(O)OR¹⁵, wherein the alkyl, aryl and heterocyclyl groups are optionally substituted by up to three groups independently selected from R¹⁷;
R¹⁰ is -OR¹⁸, C₁₋₆alkyl, -(CH₂)garyl, -(CH₂)gheterocyclyl or -(CH₂)ₕO(CH₂)ᵢOR⁷, wherein each R¹⁰ group is optionally substituted by up to three groups independently selected from R¹⁷;
R¹¹ is hydrogen or C₁₋₆alkyl;
R¹² is a heterocyclic group having the following structure: 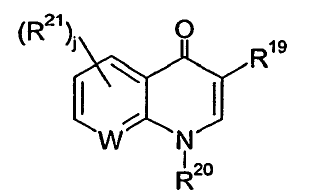 or 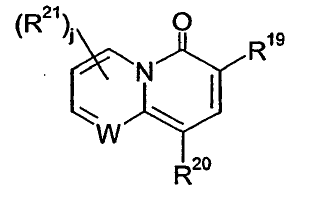 R¹³ is hydrogen or C₁₋₆alkyl;
R¹⁴ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁵ and R¹⁶ are each independently hydrogen or C₁₋₆alkyl;
R¹⁷ is halogen, cyano, nitro, trifluoromethyl, azido, -C(O)R²², -C(O)OR²², -OC(O)R²²,-OC(O)OR²², -NR²³C(O)R²⁴, -C(O)NR²³R²⁴, -NR²³R²⁴, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁵R¹⁶, halogen and -OR¹⁵, and the aryl and heteroaryl groups are optionally substituted by up to five groups independently selected from halogen, cyano, nitro, trifluoromethyl, azido,-C(O)R²⁵, -C(O)OR²⁵, -OC(O)OR²⁵, -NR²⁶C(O)R²⁷, -C(O)NR²⁶R²⁷, -NR²⁶R²⁷, hydroxy, C₁₋₆alkyl and C₁₋₆alkoxy;
R¹⁸ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁸, -S(O)ₙR²⁸, -NR²⁸R²⁹, -CONR²⁸R²⁹, halogen and cyano;
R¹⁹ is hydrogen, -C(O)OR³⁰, -C(O)NHR³⁰, -C(O)CH₂NO₂ or -C(O)CH₂SO₂R⁷;
R²⁰ is hydrogen, C₁₋₄alkyl optionally substituted by hydroxy or C₁₋₄alkoxy, C₃₋₇cycloalkyl, or optionally substituted phenyl or benzyl;
R²¹ is halogen, C₁₋₄alkyl, C₁₋₄thioalkyl, C₁₋₄alkoxy, -NH₂, -NH(C₁₋₄alkyl) or -N(C₁₋₄alkyl)₂;
R²² is hydrogen, C₁₋₁₀alkyl, -(CH₂)ₚaryl or-(CH₂)ₚheteroaryl;
R²³ and R²⁴ are each independently hydrogen, -OR¹⁵, C₁₋₆alkyl, -(CH₂)_{q}aryl or-(CH₂)_{q}heterocyclyl;
R²⁵ is hydrogen, C₁₋₁₀alkyl, -(CH₂)ᵣaryl or-(CH₂)ᵣheteroaryl;
R²⁶ and R²⁷ are each independently hydrogen, -OR¹⁵, C₁₋₆alkyl, -(CH₂)ₛaryl or-(CH₂)ₛheterocyclyl;
R²⁸ and R²⁹ are each independently hydrogen, C₁₋₄alkyl or C₁₋₄alkoxyC₁₋₄alkyl;
R³⁰ is hydrogen,
C₁₋₆alkyl optionally substituted by up to three groups independently selected from halogen, cyano, C₁₋₄alkoxy optionally substituted by phenyl or C₁₋₄alkoxy,-C(O)C₁₋₆alkyl, -C(O)OC₁₋₆alkyl, -OC(O)C₁₋₆alkyl, -OC(O)OC₁₋₆alkyl,-C(O)NR³³R³⁴, -NR³³R³⁴ and phenyl optionally substituted by nitro or -C(O)OC₁₋₆alkyl,
-(CH₂)_{w}C₃₋₇cycloalkyl,
-(CH₂)_{w}heterocyclyl,
-(CH₂)_{w}heteroaryl,
-(CH₂)_{w}aryl,
C₃₋₆alkenyl, or
C₃₋₆alkynyl;
R³¹ is hydrogen, C₁₋₄alkyl, C₃₋₇cycloalkyl, optionally substituted phenyl or benzyl, acetyl or benzoyl;
R³² is hydrogen or R²¹, or R³² and R²⁰ are linked to form the bivalent radical -O(CH₂)₂- or -(CH₂)ₜ-;
R³³ and R³⁴ are each independently hydrogen or C₁₋₆alkyl optionally substituted by phenyl or -C(O)OC₁₋₆alkyl, or
R³³ and R³⁴, together with the nitrogen atom to which they are bound, form a 5 or 6 membered heterocyclic group optionally containing one additional heteroatom selected from oxygen, nitrogen and sulfur;
X is -U(CH₂)ᵥB-, -U(CH₂)ᵥ- or a group selected from: 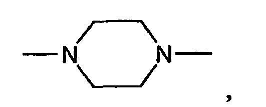 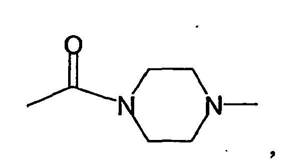 and 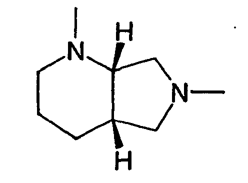 U and B are independently a divalent radical selected from -N(R³¹)-, -O-, -S(O)_{z}-,-N(R³¹)C(O)-, -C(O)N(R³¹)- and -N[C(O)R³¹]-;
W is -C(R³²)- or a nitrogen atom;
d is an integer from 2 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 1 to 8;
and pharmaceutically acceptable derivatives thereof.

According to a further embodiment the present invention provides compounds of general formula (IA) 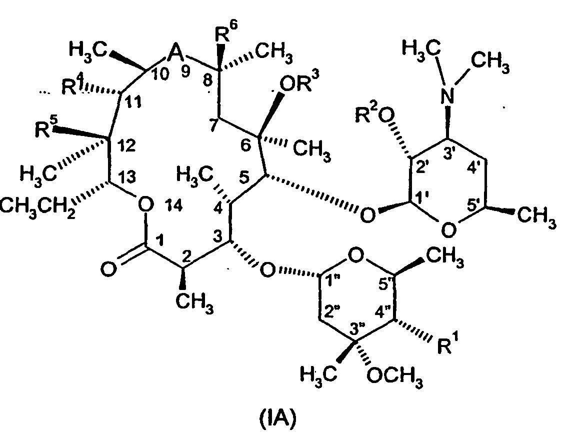 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)N(R¹¹)(CH₂)_{d}XR¹²;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹³,
R⁵ is hydroxy, or
R⁴ and R⁵ taken together with the intervening atoms form a cyclic group having the following structure: 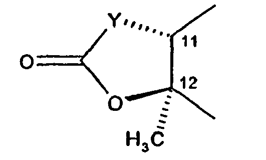 wherein Y is a bivalent radical selected from -CH₂-, -CH(CN)-, -O-, -N(R¹⁴)- and-CH(SR¹⁴)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁵R¹⁶ or-C(O)R¹⁵, or
R⁸ and R⁹ together form =CH(CR¹⁵R¹⁶)_{f}aryl, =CH(CR¹⁵R¹⁶)_{f}heterocyclyl, =CR¹⁵R¹⁶ or =C(R¹⁵)C(O)OR¹⁵, wherein the alkyl, aryl and heterocyclyl groups are optionally substituted by up to three groups independently selected from R¹⁷;
R¹⁰ is -OR¹⁸, C₁₋₆alkyl, -(CH₂)_{g}aryl, -(CH₂)gheterocyclyl or -(CH₂)ₕO(CH₂)ᵢOR⁷, wherein each R¹⁰ group is optionally substituted by up to three groups independently selected from R¹⁷;
R¹¹ is hydrogen or C₁₋₆alkyl;
R¹² is a heterocyclic group having the following structure: 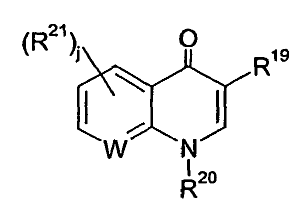 or 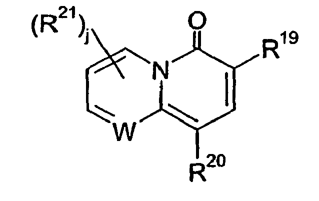 R¹³ is hydrogen or C₁₋₆alkyl;
R¹⁴ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁵ and R¹⁶ are each independently hydrogen or C₁₋₆alkyl;
R¹⁷ is halogen, cyano, nitro, trifluoromethyl, azido, -C(O)R²², -C(O)OR²², -OC(O)R²²,-OC(O)OR²², -NR²³C(O)R²⁴, -C(O)NR²³R²⁴, -NR²³R²⁴, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁵R¹⁶, halogen and -OR¹⁵, and the aryl and heteroaryl groups are optionally substituted by up to five groups independently selected from halogen, cyano, nitro, trifluoromethyl, azido,-C(O)R²⁵, -C(O)OR²⁵, -OC(O)OR²⁵, -NR²⁶C(O)R²⁷, -C(O)NR²⁶R²⁷, -NR²⁶R²⁷, hydroxy, C₁₋₆alkyl and C₁₋₆alkoxy;
R¹⁸ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁸, -S(O)ₙR²⁸, -NR²⁸R²⁹, -CONR²⁸R²⁹, halogen and cyano;
R¹⁹ is hydrogen, -C(O)OR³⁰, -C(O)NHR³⁰ or -C(O)CH₂NO₂;
R²⁰ is hydrogen, C₁₋₄alkyl optionally substituted by hydroxy or C₁₋₄alkoxy, C₃₋₇cycloalkyl, or optionally substituted phenyl or benzyl;
R²¹ is halogen, C₁₋₄alkyl, C₁₋₄thioalkyl, C₁₋₄alkoxy, -NH₂, -NH(C₁₋₄alkyl) or -N(C₁₋₄alkyl)₂;
R²² is hydrogen, C₁₋₁₀alkyl, -(CH₂)ₚaryl or -(CH₂)ₚheteroaryl;
R²³ and R²⁴ are each independently hydrogen, -OR¹⁵, C₁₋₆alkyl, -(CH₂)_{q}aryl or-(CH₂)_{q}heterocyclyl;
R²⁵ is hydrogen, C₁₋₁₀alkyl, -(CH₂)ᵣaryl or -(CH₂)ᵣheteroaryl;
R²⁶ and R²⁷ are each independently hydrogen, -OR¹⁵, C₁₋₆alkyl, -(CH₂)ₛaryl or-(CH₂)ₛheterocyclyl;
R²⁸ and R²⁹ are each independently hydrogen, C₁₋₄alkyl or C₁₋₄alkoxyC₁₋₄alkyl;
R³⁰ is hydrogen or C₁₋₆alkyl optionally substituted by up to three groups independently selected from halogen, C₁₋₄alkoxy, -OC(O)C₁₋₆alkyl and -OC(O)OC₁₋₆alkyl;
R³¹ is hydrogen, C₁₋₄alkyl, C₃₋₇cycloalkyl, optionally substituted phenyl or benzyl, acetyl or benzoyl;
R³² is hydrogen or R²¹, or R³² and R²⁰ are linked to form the bivalent radical -O(CH₂)₂- or -(CH₂)ₜ-;
X is -U(CH₂)ᵥB-, -U(CH₂)ᵥ- or a group selected from: 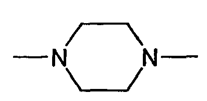 and 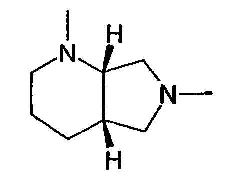 U and B are independently a divalent radical selected from -N(R³¹)-, -O-, -S(O)_{z}-,-N(R³¹)C(O)-, -C(O)N(R³¹)- and -N[C(O)R³¹]-;
W is -C(R³²)- or a nitrogen atom;
d is an integer from 2 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 2 to 8;
and pharmaceutically acceptable derivatives thereof.

The term "pharmaceutically acceptable" as used herein means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters, in particular salts.

The compounds of the present invention may be in the form of and/or may be administered as a pharmaceutically acceptable salt. For a review on suitable salts see Berge et al., J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula (I) and the resulting mixture evaporated to dryness (lyophilised) to obtain the acid addition salt as a solid. Alternatively, a compound of formula (I) may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (eg methanesulphonate, ethanesulphonate, benzenesulphonate or p-toluenesulphonate) and isethionate. Typical examples include trifluoroacetate and formate salts, for example the bis or tris trifluoroacetate salts and the mono or diformate salts. A representative salt is the monoformate salt.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Compounds of the invention may have both a basic and an acidic centre may therefore be in the form of zwitterions.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula (I) may form solvates (e.g. hydrates) and the invention also includes all such solvates..

The term "prodrugs" as used herein means a compound which is converted within the body, e.g. by hydrolysis in the blood, into its active form that has medical effects. Pharmaceutically acceptable prodrugs are described in T. Higuchi and V. Stella, "Prodrugs as Novel Delivery Systems", Vol. 14 of the A.C.S. Symposium Series, Edward B. Roche, ed., "Bioreversible Carriers in Drug Design", American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of structure (I) *in vivo* when such prodrug is administered to a patient. Prodrugs are generally prepared by modifying functional groups in a way such that the modification is cleaved, either by routine manipulation or *in vivo*, yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of structure (I). Further, in the case of a carboxylic acid (-COOH), esters may be employed, such as methyl esters, ethyl esters, and the like. Esters may be active in their own right and/or be hydrolysable under *in vivo* conditions in the human body. Suitable pharmaceutically acceptable *in vivo* hydrolysable ester groups include those which break down readily in the human body to leave the parent acid or its salt.

References hereinafter to a compound according to the invention include both compounds of formula (I) and their pharmaceutically acceptable derivatives.

With regard to stereoisomers, the compounds of structure (I) have more than one asymmetric carbon atom. In the general formula (I) as drawn, the solid wedge shaped bond indicates that the bond is above the plane of the paper. The broken bond indicates that the bond is below the plane of the paper.

It will be appreciated that the substituents on the macrolide may also have one or more asymmetric carbon atoms. Thus, the compounds of structure (I) may occur as individual enantiomers or diastereomers. All such isomeric forms are included within the present invention, including mixtures thereof.

Where a compound of the invention contains an alkenyl group, cis (Z) and trans (E) isomerism may also occur. The present invention includes the individual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C., of the corresponding mixture using a suitable chiral support or by fractional crystallisation of the diastereoisomeric salts formed by reaction of the corresponding mixture with a suitable optically active acid or base, as appropriate.

The compounds of structure (I) may be in crystalline or amorphous form. Furthermore, some of the crystalline forms of the compounds of structure (I) may exist as polymorphs, which are included in the present invention.

Compounds wherein R² represents a hydroxyl protecting group are in general intermediates for the preparation of other compounds of formula (I).

When the group OR² is a protected hydroxyl group this is conveniently an ether or an acyloxy group. Examples of particularly suitable ether groups include those in which R² is a trialkylsilyl (i.e. trimethylsilyl). When the group OR² represents an acyloxy group, then examples of suitable groups R² include acetyl or benzoyl.

R⁶ is hydrogen or fluorine. However, it will be appreciated that when A is -C(O)NH- or-CH₂-N(R⁷)-, R⁶ is hydrogen.

When R¹² is a heterocyclic group having the following structure: 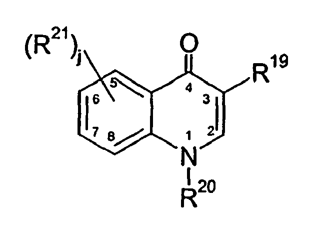 said heterocyclic is linked in the 5, 6, 7 or 8 position to the X group as above defined. In one embodiment, the heterocyclic is linked in the 6 or 7 position. In another embodiment, the heterocyclic is linked in the 5 or 8 position. When present, the R²¹ group or groups may be attached at any position on the ring. In one embodiment, an R²¹ group is attached at the 7 position.

When R¹² is a heterocyclic group having the following structure: 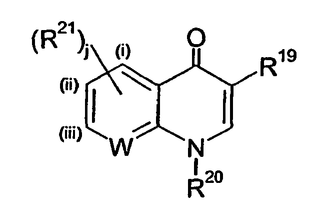 wherein W is -C(R³²)- where R³² is R²¹ or R³² and R²⁰ are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R¹² is a heterocyclic group having the following structure: 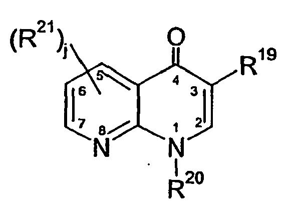 said heterocyclic is linked in the 5, 6 or 7 position to the X group as defined above. In one embodiment, the heterocyclic is linked in the 6 or 7 position. In another embodiment, the heterocyclic is linked in the 5 position.

When R¹² is a heterocyclic group having the following structure: 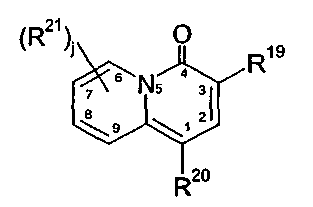 said heterocyclic is linked in the 6, 7, 8 or 9 position to the X group as above defined. In one embodiment, the heterocyclic is linked in the 7 or 8 position. In another embodiment, the heterocyclic is linked in the 6 or 9 position.

When R¹² is a heterocyclic group having the following structure: 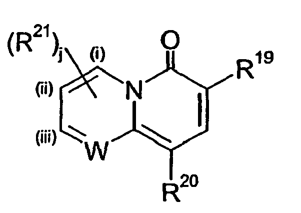 wherein W is -C(R³²)- where R³² is R²¹ or R³² and R²⁰ are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R¹² is a heterocyclic group having the following structure: 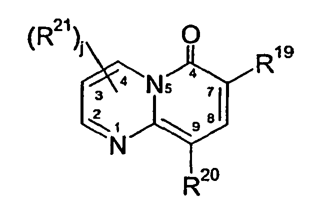 said heterocyclic is linked in the 2, 3 or 4 position to the X group as above defined. In one embodiment, the heterocyclic is linked in the 2 or 3 position. In another embodiment, the heterocyclic is linked in the 4 position.

The term "alkyl" as used herein as a group or a part of a group refers to a straight or branched hydrocarbon chain containing the specified number of carbon atoms. For example, C₁₋₁₀alkyl means a straight or branched alkyl containing at least 1, and at most 10, carbon atoms. Examples of "alkyl" as used herein include, but are not limited to, methyl, ethyl, n-propyl, n-butyl, n-pentyl, isobutyl, isopropyl, t-butyl, hexyl, heptyl, octyl, nonyl and decyl. A C₁₋₄alkyl group is preferred, for example methyl, ethyl, n-propyl, isopropyl, n-butyl, isobutyl or t-butyl.

The term "C₃₋₇cycloalkyl" group as used herein refers to a non-aromatic monocyclic hydrocarbon ring of 3 to 7 carbon atoms such as, for example, cyclopropyl, cyclobutyl, cyclopentyl, cyclohexyl or cycloheptyl.

The term "alkoxy" as used herein refers to a straight or branched chain alkoxy group containing the specified number of carbon atoms. For example, C₁₋₆alkoxy means a straight or branched alkoxy containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy and hexyloxy. A C₁₋₄alkoxy group is preferred, for example methoxy, ethoxy, propoxy, prop-2-oxy, butoxy, but-2-oxy or 2-methylprop-2-oxy.

The term "alkenyl" as used herein as a group or a part of a group refers to a straight or branched hydrocarbon chain containing the specified number of carbon atoms and containing at least one double bond. For example, the term "C₂₋₆alkenyl" means a straight or branched alkenyl containing at least 2, and at most 6, carbon atoms and containing at least one double bond. Similarly, the term "C₃₋₆alkenyl" means a straight or branched alkenyl containing at least 3, and at most 6, carbon atoms and containing at least one double bond. Examples of "alkenyl" as used herein include, but are not limited to, ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl. It will be appreciated that in groups of the form -O-C₂₋₆alkenyl, the double bond is preferably not adjacent to the oxygen.

The term "alkynyl" as used herein as a group or a part of a group refers to a straight or branched hydrocarbon chain containing the specified number of carbon atoms and containing at least one triple bond. For example, the term "C₃₋₆alkenyl" means a straight or branched alkynyl containing at least 3, and at most 6, carbon atoms containing at least one triple bond. Examples of "alkynyl" as used herein include, but are not limited to, propynyl, 1-butynyl, 2-butynyl, 1-pentynyl and 3-methyl-1-butynyl.

The term "aryl" as used herein refers to an aromatic carbocyclic moiety such as phenyl, biphenyl or naphthyl.

The term "heteroaryl" as used herein, unless otherwise defined, refers to an aromatic heterocycle of 5 to 10 members, having at least one heteroatom selected from nitrogen, oxygen and sulfur, and containing at least 1 carbon atom, including both mono and bicyclic ring systems. Examples of heteroaryl rings include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, thiadiazolyl, pyridyl, pyridazinyl, pyrazinyl, pyrimidinyl, triazinyl, quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl and benzothiophenyl.

The term "5 or 6 membered heteroaryl" as used herein as a group or a part of a group refers to a monocyclic 5 or 6 membered aromatic heterocycle containing at least one heteroatom independently selected from oxygen, nitrogen and sulfur. Examples include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, pyridyl, pyridazinyl, pyrazinyl, pyrimidinyl and triazinyl.

The term "9 to 10 membered fused bicyclic heteroaryl" as used herein as a group or a part of a group refers to quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl or benzothiophenyl.

The term "heterocyclyl" as used herein, unless otherwise defined, refers to a monocyclic or bicyclic three- to ten-membered saturated or non-aromatic, unsaturated hydrocarbon ring containing at least one heteroatom selected from oxygen, nitrogen and sulfur. Preferably, the heterocyclyl ring has five or six ring atoms. Examples of heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "5 or 6 membered heterocyclic group" as used herein as a group or part of a group refers to a monocyclic 5 or 6 membered saturated hydrocarbon ring containing at least one heteroatom independently selected from oxygen, nitrogen and sulfur. Examples of such heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "halogen" refers to a fluorine, chlorine, bromine or iodine atom.

The terms "optionally substituted phenyl", "optionally substituted phenyl or benzyl", "optionally substituted 5 or 6 membered heteroaryl", "optionally substituted 9 to 10 membered fused bicyclic heteroaryl, or "optionally substituted 5 or 6 membered heterocyclic group" as used herein refer to a group which is substituted by 1 to 3 groups selected from halogen, C₁₋₄alkyl, C₁₋₄alkoxy, hydroxy, nitro, cyano, amino, C₁₋₄alkylamino or diC₁₋₄alkylamino, phenyl and 5 or 6 membered heteroaryl.

In one embodiment, A is -C(O)-, -C(O)NH-, -NHC(O)-, -N(R⁷)CH₂-, -CH₂-N(R⁷)- or-CH(NR⁸R⁹)-. In another embodiment, A is -C(O)-, -C(O)NH-, -NHC(O)-, -CH₂-N(R⁷)-,-CH(NR⁸R⁹)- or -C(=NR¹⁰)-. In a further embodiment, A is -C(O)-, -C(O)NH-, -NHC(O)-,-CH₂-NR⁷- or -CH(NR⁸R⁹)-. Representative examples of A include -C(O)- and -N(R⁷)-CH₂-.

A representative example of R² is hydrogen.

Representative examples of R³ include hydrogen and C₁₋₄alkyl, in particular hydrogen and methyl.

In one embodiment, R⁴ and R⁵ are hydroxy, or R⁴ and R⁵ taken together with the intervening atoms form a cyclic group having the following structure: 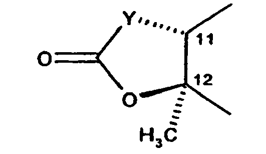 wherein Y is a bivalent radical selected from -CH₂-, -CH(CN)-, -O-, -N(R¹⁴)- and-CH(SR¹⁴)-. In another embodiment, R⁴ is hydroxy or C₁₋₆alkoxy, in particular methoxy, and R⁵ is hydroxy. A representative example of R⁴ and R⁵ is hydroxy. A further representative example of R⁴ is C₁₋₄alkoxy, in particular methoxy.

A representative example of R⁶ is hydrogen.

A representative example of R⁷ is C₁₋₆alkyl, for example C₁₋₄alkyl, in particular methyl.

A representative example of R¹¹ is hydrogen.

Representative examples of R¹² include heterocyclic groups having the following structure: 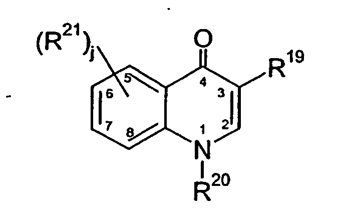 wherein the heterocyclic is linked in the 6 or 7 position to the X group as above defined. In particular, the heterocyclic is linked in the 6 position.

In one embodiment, R¹⁴ is hydrogen or C₁₋₄alkyl substituted by a group selected from optionally substituted phenyl, optionally substituted 5 or 6 membered heteroaryl and optionally substituted 9 to 10 membered fused bicyclic heteroaryl.

In one embodiment, R¹⁹ is hydrogen, -C(O)OR³⁰, -C(O)NHR³⁰ or -C(O)CH₂NO₂. In a further embodiment, R¹⁹ is -C(O)OR³⁰, -C(O)NHR³⁰⁹ or -C(O)CH₂NO₂. A representative example of R¹⁹ is -C(O)OR³⁰, wherein R³⁰ is hydrogen.

Representative examples of R²⁰ include C₁₋₄alkyl, in particular ethyl, and C₃₋₇cycloalkyl, in particular cyclopropyl.

In one embodiment, R²¹ is halogen, in particular chlorine.

In one embodiment, R³⁰ is hydrogen or C₁₋₆alkyl optionally substituted by up to three groups independently selected from halogen, C₁₋₄alkoxy, -OC(O)C₁₋₆alkyl and-OC(O)OC₁₋₆alkyl. A representative example of R³⁰ is hydrogen.

In one embodiment, R³¹ is hydrogen or C₁₋₄alkyl. A representative example of R³¹ is hydrogen. A further representative example of R³¹ is C₁₋₄alkyl, in particular methyl.

A representative example of R³² is hydrogen.

In one embodiment, X is -U(CH₂)ᵥB-, -U(CH₂)ᵥ- or a group selected from: 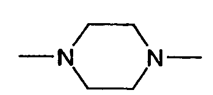 and

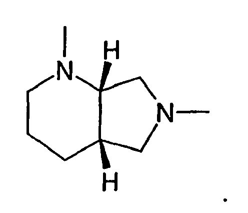

Representative examples of X are -U(CH₂)ᵥB- and -U(CH₂)ᵥ-.

In one embodiment, U and B are independently a divalent radical selected from -N(R³¹)-, -O- and -S(O)_{z}-. Representative examples of U and B include the divalent radicals-N(R³¹)- and -O-. A further representative example of U and B is -S(O)_{z}-.

In one embodiment, when X is -U(CH₂)ᵥB-, U is selected from the divalent radicals-N(R³¹)- and -O-, and B is selected from the divalent radicals -N(R³¹)-, -O- and -S(O)₂-. In particular, U is selected from the divalent radicals -N(R³¹)- and -O-, and B is the divalent radical -O-. Alternatively, U is selected from the divalent radicals -N(R³¹)- and-O-, and B is selected from the divalent radicals -N(R³¹)- and -S(O)_{z}-.

In one embodiment, when X is -U(CH₂)ᵥ-, U is selected from the divalent radicals-N(R³¹)- and -O-.

A representative example of d is 1 to 3, for example 2 or 3.

In one embodiment, v is an integer from 2 to 8. In another embodiment, v is an integer from 1 to 4. A representative example of v is 2 to 4, for example 2 or 3. A further representative example of v is 1.

Representative examples of j include 0 and 1. In particular, j is 0.

A representative example of z is 0.

It is to be understood that the present invention covers all combinations of particular and preferred groups described hereinabove. It is also to be understood that the present invention encompasses compounds of formula (I) in which a particular group or parameter, for example R⁷, R¹⁵, R¹⁶, R¹⁷ R²¹, R²², R²³, R²⁴, R²⁵, R²⁶, R²⁷, R²⁸, R²⁹, R³¹, R³³, R³⁴ k, m, n, p, q, r , s and z may occur more than once. In such compounds it will be appreciated that each group or parameter is independently selected from the values listed.

Particularly preferred compounds of the invention are:
4"-O-3-[2-(3-carboxy-7-chloro-1-cyclopropyl-4-oxo-1,4-dihydro-quinolin-6-yloxy)-ethoxy]-propylcarbamoyloxy}-azithromycin;
4"-O-{3-[2-(3-carboxy-1-cyclopropyl-4-oxo-1,4-dihydro-quinolin-6-yloxy)-ethoxy]-propylcarbamoyloxy}-azithromycin;
and pharmaceutically acceptable derivatives thereof.

Further particularly preferred compounds of the invention are:
4"-*O*-{2-[3-(3-carboxy-1-ethyl-4-oxo-1,4-dihydro-quinolin-6-yl)-propyloxy]-ethylcarbamoyl}-6-*O*-methyl-erythromycin A;
4"-*O*-{3-[2-(3-carboxy-1-cyclopropyl-4-oxo-1,4-dihydro-quinolin-6-ylamino)-ethoxy]-propylcarbamoyl}-azithromycin;
4"-*O*-{3-[2-(3-carboxy-1-cyclopropyl-4-oxo-1,4-dihydro-quinolin-6-yloxy)-ethoxy]-propylcarbamoyl}-11-*O*-methylazithromycin;
4"-*O*-{3-[2-(3-carboxy-1-cyclopropyl-4-oxo-1,4-dihydro-quinolin-6-ylamino)-ethoxy]-propylcarbamoyl}-11-*O*-methylazithromycin;
and pharmaceutically acceptable derivatives thereof.

Compounds according to the invention also exhibit a broad spectrum of antimicrobial activity, in particular antibacterial activity, against a wide range of clinical pathogenic microorganisms. Using a standard microtiter broth serial dilution test, compounds of the invention have been found to exhibit useful levels of activity against a wide range of pathogenic microorganisims. In particular, the compounds of the invention may be active against strains of Staphylococcus aureus, Streptopococcus pneumoniae, Moraxella catarrhalis, Streptococcus pyogenes, Haemophilus influenzae, Enterococcus faecalis, Chlamydia pneumoniae, Mycoplasma pneumonia and Legionella pneumophila. The compounds of the invention may also be active against resistant strains, for example erythromycin resistant strains. In particular, the compounds of the invention may be active against erythromycin resistant strains of Streptococcus pneumoniae, Streptococcus pyogenes and Staphylococcus aureus.

The compounds of the invention may therefore be used for treating a variety of diseases caused by pathogenic microorganisms, in particular bacteria, in human beings and animals. It will be appreciated that reference to treatment includes acute treatment or prophylaxis as well as the alleviation of established symptoms.

Thus, according to another aspect of the present invention we provide a compound of formula (I) or a pharmaceutically acceptable derivative thereof for use in therapy.

According to a further aspect of the invention we provide a compound of formula (I) or a pharmaceutically acceptable derivative thereof for use in the therapy or prophylaxis of systemic or topical microbial infections in a human or animal subject.

According to a further aspect of the invention we provide the use of a compound of formula (I) or a pharmaceutically acceptable derivative thereof in the manufacture of a medicament for use in the treatment or prophylaxis of systemic or topical microbial infections in a human or animal body.

According to a yet further aspect of the invention we provide a method of treatment of the human or non-human animal body to combat microbial infections comprising administration to a body in need of such treatment of an effective amount of a compound of formula (I) or a pharmaceutically acceptable derivative thereof.

While it is possible that, for use in therapy, a compound of the invention may be administered as the raw chemical it is preferable to present the active ingredient as a pharmaceutical formulation eg when the agent is in admixture with a suitable pharmaceutical excipient, diluent or carrier selected with regard to the intended route of administration and standard pharmaceutical practice.

Accordingly, in one aspect, the present invention provides a pharmaceutical composition or formulation comprising at least one compound of the invention or a pharmaceutically acceptable derivative thereof in association with a pharmaceutical acceptable excipient, diluent and/or carrier. The excipient, diluent and/or carrier must be "acceptable" in the sense of being compatible with the other ingredients of the formulation and not deleterious to the recipient thereof.

In another aspect, the invention provides a pharmaceutical composition comprising, as active ingredient, at least one compound of the invention or a pharmaceutically acceptable derivative thereof in association with a pharmaceutically acceptable excipient, diluent and/or carrier for use in therapy, and in particular, in the treatment of human or animal subjects suffering from a condition susceptible to amelioration by an antimicrobial compound.

In another aspect, the invention provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable derivative thereof, together with a pharmaceutically acceptable excipient, diluent and/or carrier.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable excipients, diluents and/or carriers. Acceptable excipients, diluents and carriers for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent and/or carrier can be selected with regard to the intended route of administration and standard pharmaceutical practice. The pharmaceutical compositions may comprise as - or in addition to - the excipient, diluent and/or carrier any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

There may be different composition/formulation requirements depending on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

It is to be understood that not all of the compounds need be administered by the same route. Likewise, if the composition comprises more than one active component, then those components may be administered by different routes.

The compositions of the invention include those in a form especially formulated for parenteral, oral, buccal, rectal, topical, implant, ophthalmic, nasal or genito-urinary use. For some applications, the agents of the present invention are delivered systemically (such as orally, buccally, sublingually), more preferably orally. Hence, preferably the agent is in a form that is suitable for oral delivery.

If the compound of the present invention is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques.

For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention can be administered (e. g. orally or topically) in the form of tablets, capsules, ovules, elixirs, solutions or suspensions, which may contain flavouring or colouring agents, for immediate-, delayed-, modified-, sustained-, pulsed- or controlled-release applications.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably com, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (e.g. eye ear or nose drops) or pour-ons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds may also be dermally or transdermally administered, for example, by use of a skin patch.

For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

As indicated, the compound of the present invention can be administered intranasally or by inhalation and is conveniently delivered in the form of a dry powder inhaler or an aerosol spray presentation from a pressurised container, pump, spray or nebuliser with the use of a suitable propellant, e. g. dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e. g. using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebuliser.

The compounds of the invention may also be used in combination with other therapeutic agents. The invention thus provides, in a further aspect, a combination comprising a compound of the invention or a pharmaceutically acceptable derivative thereof together with a further therapeutic agent.

When a compound of the invention or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian. The compounds of the present invention may for example be used for topical administration with other active ingredients such as corticosteroids or antifungals as appropriate.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

The compositions may contain from 0.01-99% of the active material. For topical administration, for example, the composition will generally contain from 0.01-10%, more preferably 0.01-1% of the active material.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses.

For systemic administration the daily dose as employed for adult human treatment it will range from 2-100mg/kg body weight, preferably 5-60mg/kg body weight, which may be administered in 1 to 4 daily doses, for example, depending on the route of administration and the condition of the patient. When the composition comprises dosage units, each unit will preferably contain 200mg to 1g of active ingredient. The duration of treatment will be dictated by the rate of response rather than by arbitrary numbers of days.

Compounds of general formula (I) and salts thereof may be prepared by the general methods outlined hereinafter, said methods constituting a further aspect of the invention. In the following description, the groups R¹ to R³⁴, A, B, X, Y, U, W, d, e, f, g, h, i, j, k, m, - n, p, q, r, s, t, v, w and z have the meaning defined for the compounds of formula (I) unless otherwise stated.

The group X^{a}R^{12a} is XR¹² as defined for formula (I) or a group convertible to XR¹². Conversion of a group X^{a}R^{12a} to a XR¹² group typically arises if a protecting group is needed during the reactions described below. A comprehensive discussion of the ways in which such groups may be protected and methods for cleaving the resulting protected derivatives is given by for example T.W. Greene and P.G.M Wuts in Protective Groups in Organic Synthesis 2nd ed., John Wiley & Son, Inc 1991 and by P.J. Kocienski in Protecting Groups, Georg Thieme Verlag 1994 which are incorporated herein by reference. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (F_{moc})), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tert-butyldimethylsilyl; alkyl ethers such as tetrahydropyranyl or tert-butyl; or esters such as acetate. Hydroxy groups may be protected by reaction of for example acetic anhydride, benzoic anhydride or a trialkylsilyl chloride in an aprotic solvent. Examples of aprotic solvents are dichloromethane, N,N-dimethylformamide, dimethylsulfoxide, tetrahydrofuran and the like.

Compounds of formula (I) may be prepared by reaction of a suitable activated compound of formula (II) wherein R² is optionally a hydroxy protecting group and R³⁵ is an activating group such as imidazolyl or halogen, with a suitable protected derivative of the amine (III), followed where necessary by subsequent removal of the hydroxyl protecting group R² and conversion of the X^{a}R^{12a} group to XR¹².

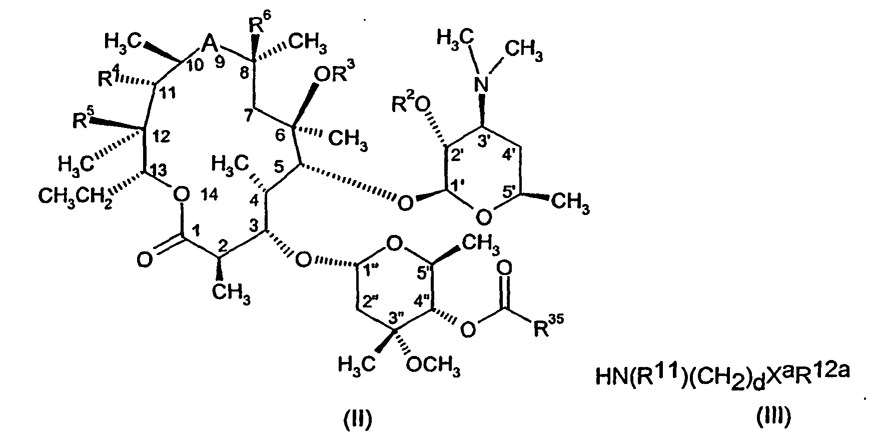

The reaction is preferably carried out in a suitable aprotic solvent such as N,N-dimethylformamide in the presence of an organic base such as 1,8-diazabiyclo[5.4.0]undec-7-ene (DBU).

In a further embodiment of the invention, compounds of formula (I) wherein U is a group selected from -N(R³¹)- and -S-, may be prepared by reaction of compounds of formula (IV) 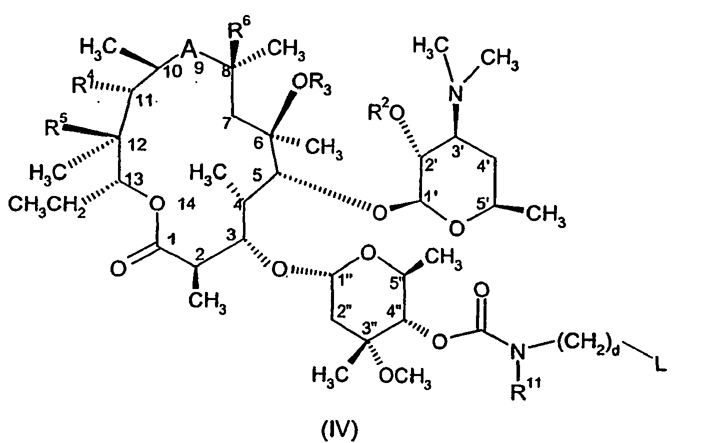 wherein d is an integer from 2 to 5 and L is a suitable leaving group, with X^{a}R^{12a} (V) in which U is a group selected from -N(R³¹)- and -S-. The reaction is preferably carried out in a solvent such as a halohydrocarbon (e.g. dichloromethane), an ether (e.g. tetrahydrofuran or dimethoxyethane), acetonitrile or ethyl acetate and the like, dimethylsulfoxide, N,N-dimethylformamide or 1-methyl-pyrrolidone and in the presence of a base, followed, if desired, by removal of the hydroxyl protecting group R² and conversion of the X^{a}R^{12a} group to XR¹². Examples of the bases which may be used include organic bases such as diisopropylethylamine, triethylamine and 1,8-diazabicyclo[5.4.0]undec-7-ene (DBU), and inorganic bases such as potassium hydroxide, cesium hydroxide, tetraalkylammonium hydroxide, sodium hydride, potassium hydride and the like. Suitable leaving groups for this reaction include halide (e.g. chloride, bromide or iodide) or a sulfonyloxy group (e.g. tosyloxy or methanesulfonyloxy).

Compounds of formula (I) may be converted into other compounds of formula (I). Thus compounds of formula (I) wherein U or B is -S(O)₂- and z is 1 or 2 may be prepared by oxidation of the corresponding compound of formula (I) wherein z is 0. The oxidation is preferably carried out using a peracid, e.g. peroxybenzoic acid, followed by treatment with a phosphine, such as triphenylphosphine. The reaction is suitably carried out in an organic solvent such as methylene chloride. Compounds of formula (I) wherein U is -N(R³¹)- and R³¹ is C₁₋₄alkyl can be prepared from compounds wherein R³¹ is hydrogen by reductive alkylation.

Compounds of formula (II) wherein A is -C(O)NH- or -NHC(O)-, R⁴ or R⁵ are hydroxy, R³ is hydrogen and R⁶ is hydrogen are known compounds or they may be prepared by analogous methods to those known in the art. Thus they can be prepared according to the procedures described in EP 507595 and EP 503932.

Compounds of formula (II), wherein A is -C(O)NH- or -NHC(O)-, R⁴ or R⁵ are hydroxy and R³ is C₁₋₄alkyl or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in WO 9951616 and WO 0063223.

Compounds of formula (II), wherein A is -C(O)NH-, R⁴ and R⁵ taken together with the intervening atoms form a cyclic group having the following structure:

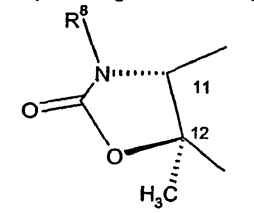

R³ is C₁₋₄alkyl, or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in US 6262030.

Compounds of formula (II), wherein A is -C(O)-, -C(O)NH-, -NHC(O)-, -N(R⁷)-CH₂-, -CH₂-N(R⁷)- or -CH(NR⁸R⁹)-, R⁴ or R⁵ are hydroxy or R⁴ and R⁵ taken together with the intervening atoms form a cyclic group having the following structure: 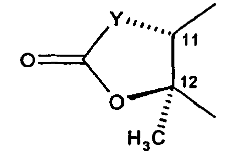 wherein Y is a bivalent radical selected from -O- and -N(R¹³)-, and R³ is C₁₋₄alkyl, or C₃₋₆alkenyl optionally substituted by 9 to 10 membered fused bicyclic heteroaryl are know compounds or they may be prepared by analogous methods to those known in the art. Thus they can be prepared according to the procedures described in EP 307177, EP 248279, WO 0078773, WO 9742204.

Compounds of formula (II), wherein A is -C(O)NH-, -NHC(O)-, -N(CH₃)-CH₂- or -CH₂-N(CH₃)-, R⁴ or R⁵ are hydroxy or R⁴ and R⁵ taken together with the intervening atoms form a cyclic group having the following structure: 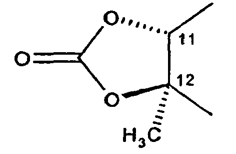 and R⁶ is hydrogen are known compounds or they may be prepared by analogous methods to those known in the art. Thus they can be prepared according to the procedures described in EP 508699 and J.Chem. Res.Synop (1988 pages 152-153), US 6262030.

Compounds of formula (II), wherein A is -C(=NR¹⁰)-, R⁴ or R⁵ are hydroxy or R⁴ and R⁵ taken together with the intervening atoms form a cyclic group having the following structure: 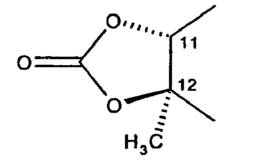 and R⁶ is hydrogen, are known compounds or they may be prepared by analogous methods to those known in the art. Thus they can be prepared according to the procedures described in EP 284203.

Compounds of formula (II), wherein A is -C(O)-, R⁴ and R⁵ taken together with the intervening atoms form a cyclic group having the following structure:

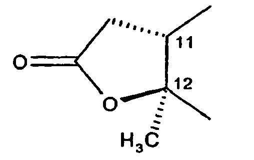

R⁶ is hydrogen and R³ is C₁₋₄ alkyl may be prepared by decarboxylation of a compound of formula (VI), wherein R³⁵ is amino protecting group followed, if required, by removal of the protecting group R² or R³⁵.

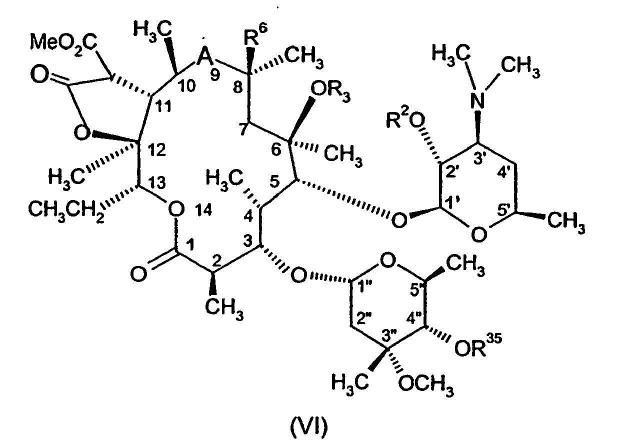

The decarboxylation may be carried out in the presence of a lithium salt such as lithium chloride, preferably in an organic solvent such as dimethylsulfoxide.

Compounds of formula (II), wherein A is -C(O)-, R⁴ and R⁵ taken together with the intervening atoms form a cyclic group having the following structure: 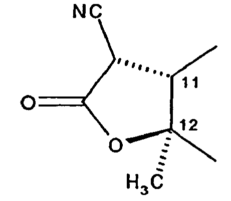 and R₃ is C₁₋₄ alkyl may be prepared according to the procedures described in WO 02/50091 and WO 02/50092.

In order that the invention may be more fully understood the following examples are given by way of illustration only.

The following abbreviations are used in the text: Ac for acetyl, BOC for t-butoxycarbonyr, DBU for 1,8diazabicyclo[5.4.0]undec-7-ene, DCM for dichloromethane, DMF for N,N-dimethylformamide, DMSO for dimethyl sulfoxide, EtOAc for ethyl acetate, EtOH for ethanol, iPrOH for isopropanol, KO^{t}Bu for potassium *tert*-butoxide, MDAP for mass-directed autopreparative HPLC, MeCN for acetonitrile, MeOH for methanol and NBS for N-bromosuccinimide.

### Examples

### 2'-O-Acetyl-azithromycin may be prepared by the procedure described by S. Djokic et al. in J. Chem. Res. (S) 1988, 152.

### Intermediate 1: 7-Chloro-1-cyclopropyl-6-(2-hydroxy-ethoxy)-4-oxo-14-dihydro-guinoline-3-carboxylic acid (A) and 1-Cyclopropyl-6-fluoro-7-(2-hydroxy-ethoxy)-4-oxo-1,4-dihydro-quinoline-3-carboxylic acid (B)

To a mixture of DMSO (5 mL) and ethyleneglycol (6 mL), KO^{t}Bu (1.6 g, 14.23 mmol) was added portionwise over 10 min, and then heated to 90 °C. To the mixture, 7-chloro-1-cyclopropyl-6-fluoro-4-oxo-1,4-dihydro-quinoline-3-carboxylic acid (1.0 g) was added portionwise over 20 min, the temperature was increased to 105 °C and the mixture was stirred for 6 h. Water (30 mL) was added to the reaction solution and the pH of the solution was adjusted to pH=5. The resulting solution was left in the refrigerator overnight. The precipitate obtained was filtered, washed with cold water, and dried affording a 2:1 mixture of **Intermediate 1A and Intermediate 1B** (1.0 g).

Part of the crude product (700 mg) was dissolved in EtOH (15 mL) by heating to the reflux. The resulting solution was cooled to 30°C and a first precipitation occurred. The precipitate was filtered, washed with cold EtOH and dried under reduced pressure. **Intermediate 1A** (204 mg) was obtained as a white solid; ¹H-NMR (500 MHz, DMSO-d6) δ: 15.06 (s, 1H), 8.71 (s, 1H), 8.40 (s, 1H), 7.86 (s, 1H), 4.97 (t, 1H), 4.25 (t, 2H), 3.87 (m, 1H), 3.82 (q, 2H), 1.32 (m, 2H), 1.20 (m, 2H). ¹³C-NMR (75 MHz, DMSO-d6) δ: 176.61, 165.67, 152.47, 147.54, 135.34, 129.48, 124.95, 120.02, 106.90, 106.66, 71.22, 59.15, 35.99, 7.46. MS; m/z (ES): [MH]⁺.

### Intermediate 2: 7-Chloro-6-[2-(2-cyano-ethoxy)-ethoxy]-1-cyclopropyl-4-oxo-1,4-dihydro-quinoline-3-carboxylic acid

To a suspension of **Intermediate 1A** (2 g) in acrylonitrile (40 mL) was added DBU (2.3 mL). The reaction mixture was stirred at 80°C for 24 h. The acrylonitrile was evaporated under reduced pressure. Isopropanol (30 mL) was added to the residue and the pH of the solution was adjusted to pH=5 by adding 2M HCL, during which the product precipitated. The precipitate was filtered, washed with water, and dried affording the title compound (1.7 g), as a white solid; ¹H-NMR (500 MHz, DMSO-d6) δ: 8.68 (s, 1H), 8.38 (s, 1H), 7.84 (s, 1H), 4.38 (t, 2H), 3.91 (t, 2H), 3.86 (m, 1H), 3.75 (t, 2H), 2.79 (t, 2H), 1.32 (m, 2H), 1.20 (m, 2H). ¹³C-NMR (75 MHz, DMSO-d6) δ: 176.63, 165.65, 152.18, 147.61, 135.50, 129.44, 124.97, 120.04, 119.11, 106.96, 106.80, 69.02, 68.30, 65.49, 35.99, 18.06, 7.46. MS; m/z (ES): 377.03 [MH]⁺.

### Intermediate 3: 6-[2-(3-Amino-propoxy)-ethoxy]-7-chloro-1-cyclopropyl-4-oxo-1,4-dihydro-quinoline-3-carboxylic acid (A) and 6-[2-(3-amino-propoxy)-ethoxy]-1-cyclopropyl-4-oxo-1,4-dihydro-quinoline-3-carboxylic acid (B)

A high pressure reactor was filled with a mixture of 0.5g (1.3 mmol) **Intermediate 1**, 40 mL of acetic acid (96%) and 0.170g PtO₂ at a H₂ pressure of 4.7 bar for 24 hours. The reaction mixture was filtered through celite and the acetic acid was evaporated *in vacuo.* Crude product was precipitated from CH₂Cl₂-diisopropyl ether yielding 0.6g of the title compounds. LC-MS showed that the product ratio is 2:1 for **3A:3B;** MS; m/z (ES): 381.03 [MH]⁺, 347.08 [MH]⁺

### Intermediate 4: 6-(2-Amino-ethoxy)-1-ethyl-4-oxo-1,4-dihydro-guinoline-3-carboxylic acid hydrochloride

### a) 6-(2-Dibenzylamino-ethoxy)-1-ethyl-4-oxo-1,4-dihydro-quinoline-3-carboxylic acid 2-dibenzylamino-ethyl ester.

1-Ethyl-6-hydroxy-4-oxo-1,4-dihydro-quinoline-3-carboxylic acid (GB 1433774) (1.4 g, 6 mmol) was dissolved in dry DMF (80 mL). To this was added potassium carbonate (5 g, 36 mmol) and dibenzyl-(2-chloroethyl)amine hydrochloride (4.37 g, 14.8 mmol). The mixture was heated at 65°C with stirring for 72 h, then allowed to cool overnight. The mixture was evaporated to a small volume, diluted with water and extracted with ethyl acetate (x2). The combined organic extracts were washed with brine, dried and evaporated under reduced pressure to give a dark viscous oil (4.9 g). This residue was purified by chromatography on silica gel (100 g), eluting with 0.2 - 3.8% methanol in dichloromethane, to give the title compound as a brown solid (2.46 g, 60%); ESMS m/z 680 [M+H]⁺ (100%).

### b) 6-(2-Dibenzylamino-ethoxy)-1-ethyl-4-oxo-1,4-dihydro-quinoline-3-carboxylic acid sodium salt.

**Intermediate 4a** (2.44 g, 3.59 mmol) was dissolved in methanol (25 mL) and 1,4-dioxane (25 mL), then aqueous sodium hydroxide (0.4N, 8.75 mL, 3.5 mmol) was added. Stirred for 40 h then a little more sodium hydroxide was added and stirring continued for a further 72 h. Excess solid carbon dioxide was then added and the mixture evaporated to dryness under reduced pressure. Trituration with diethyl ether gave the title compound as a pale brown powder (1.382 g, 84%); ESMS m/z 457 [M+H]⁺for the free acid (100%).

### c) 6-(2-Amino-ethoxy)-1-ethyl-4-oxo-1,4-dihydro-quinoline-3-carboxylic acid.

**Intermediate 4b** (1.38 g, 2.89 mmol) was dissolved in 1,4-dioxane (80 mL), water (40 mL) and hydrochloric acid (2N, 2.9 mL, 5.8 mmol). This solution was hydrogenated over 20% palladium(II) hydroxide on carbon (0.6 g) at 50 psi for 18 h. The mixture was filtered through kieselguhr, washing well with water. The filtrate was then evaporated to dryness under reduced pressure to give the title compound as a pale yellow solid (1 g, 94%) (containing one equivalent of sodium chloride); ESMS m/z 277 [M+H]⁺ for free acid (100%).

### Intermediate 5: 6-[2-(2-Amino-ethylamino)-ethoxy]-1-ethyl-4-oxo-1,4-dihydroquinoline-3-carboxylic acid bis trifluoroacetate

### a) 6-[2-(2-tert-Butoxycarbonytamino-ethylamino)-ethoxy]-1-ethyl-4-oxo-1,4-dihydroquinoline-3-carboxylic acid mono formate.

**Intermediate 4** (0.148 g, 0.4 mmol) was suspended in methanol (2 mL), DMF (2 mL), and acetic acid (0.2 mL) then sodium acetate (0.033 g, 0.4 mmol) added followed by *tert*-butyt (2-oxoethyl)carbamate (0.064 g, 0.4 mmol). This mixture was stirred for 10 min then sodium cyanoborohydride (0.050 g, 0.8 mmol) added. The reaction was stirred for 29.5 h then more sodium cyanoborohydride (0.050 g, 0.8 mmol) and DMF (2 mL) were added. After a further 15 h the mixture was evaporated to dryness. The residue was suspended in DMSO, filtered, and purified by preparative reverse phase HPLC (MeCN/H₂O/0.1%HCO₂H eluent) to give the title compound; ESMS *m*/*z* 420 [M+H]⁺.

### b) 6-[2-(2-Amino-ethylamino)-ethoxy]-1-ethyl-4-oxo-1,4-dihydro-quinoline-3-carboxylic acid bis trifluoroacetate.

**Intermediate 5a** (0.021 g, 0.045 mmol) was dissolved in DCM (4 mL), and trifluoroacetic acid (1 mL) added. The solution was stirred for 2 h then evaporated to dryness to give the title compound; ESMS *m*/*z* 320 [M+H]⁺.

### Intermediate 6: 6-[3-(2-Amino-ethylamino)-propyl]-1-ethyl-4-oxo-1,4-dihydroquinoline-3-carboxylic acid bis trifluoroacetate

### a) 1-Ethyl-6-iodo-4-oxo-1,4-dihydro-quinoline-3-carboxylic acid ethyl ester.

A mixture of 1,4-dihydro-6-iodo-4-oxo-quinoline-3-carboxylic acid (J. Ellis, E. Gellert, J. Robson, Aust. J. Chem., 1973, 26, 907) (3.15 g, 10 mmol), potassium carbonate (6.9 g, 50 mmol) and iodoethane (15.6 g, 100 mmol) in dry DMF was heated at 70°C with vigorous stirring. After 16 h the mixture was cooled and diluted with ethyl acetate. The resultant mixture was washed with water and the organic phase separated, dried and evaporated to yield the title compound as a pale yellow solid; ¹H NMR δ (CDCl₃) 1.41 (3H, t, J = 7.1 Hz), 1.54 (3H, t, J = 7.3 Hz), 4.23 (2H, q, J = 7.2 Hz), 4.40 (2H, q, J = 7.1 Hz), 7.20 (1H, d, J = 8.9 Hz), 7.95 (1H, dd, J = 2.1 & 8.9 Hz), 8.48 (1H, s), 8.86 (1H, d, J = 2.1 Hz).

### b) 6-(3-t-Butoxycarbonylamino-prop-1-ynyl)-1-ethyl-4-oxo-1,4-dihydro-quinoline-3-carboxylic acid ethyl ester.

A mixture of **Intermediate 6a** (6.89 g, 18.6 mmol) and copper (I) iodide suspended in acetonitrile (200 mL) and triethylamine (93 mL) was heated to 50°C and deoxygenated with argon for 20 min. The deep green solution was then treated with *N*-*t*-butoxycarbonyl propargylamine (5.2 g, 33.4 mmol) and bis(triphenylphosphine)palladium (II) dichloride (0.42 g, 0.6 mmol) and heated under reflux. After 2 h the mixture was cooled, filtered and evaporated to dryness, the residue was partitioned between water (250 mL) and dichloromethane (250 mL). Separation of the organic layer followed by drying and evaporation gave the crude compound which was purified by chromatography over silica gel eluting with dichloromethane containing an increasing concentration of methanol (0-6%) to yield the title compound as a yellow solid; ESMS *m*/*z* 399 [M+H]⁺.

### c) 6-(3-t-Butoxycarbonylaminopropyl)-1-ethyl-4-oxo-1,4-dihydro-quinoline-3-carboxylic acid ethyl ester.

A mixture of **Intermediate 6b** (8.0 g, 20.0 mmol) and 10% palladium on charcoal (0.5 g) in dichloromethane (200 mL) was hydrogenated at 14 psi and room temperature. After 18 h the mixture was filtered and the solvent evaporated to yield the title compound as a yellow foam; ESMS *m*/*z* 403 [M+H]⁺.

### d) 6-(3-Aminopropyl)-1-ethyl-4-oxo-1,4-dihydro-quinoline-3-carboxylic acid ethyl ester trifluoroacetate salt.

A solution of **Intermediate 6c** (0.60 g, 1.49 mmol) was treated with trifluoroacetic acid (2 mL). After stirring at room temperature for 10 mins the brown mixture was evaporated, redissolved in dichloromethane and re-evaporated. The resultant gum was triturated with water (15 mL) cooled to 5°C and filtered to yield the title compound a cream-coloured solid; ESMS *m*/*z* 303 [M+H]⁺.

### e) 6-[3-(2-tert-Butoxycarbonylamino-ethylamino)-propyl]-1-ethyl-4-oxo-1,4-dihydroquinoline-3-carboxylic acid ethyl ester.

**Intermediate 6d** (0.621 g, 1.49 mmol) dissolved in 1% acetic acid/methanol (20 mL) was treated with *tert*-butyl (2-oxoethyl)carbamate (0.24 g, 1.49 mmol) and sodium acetate (0.49 g, 5.96 mmol). The mixture was stirred at room temperature for 0.5 h then treated with sodium cyanoborohydride (0.19 g, 2.98 mmol). Over a period of 72 h a further 2 equivalents of aldehyde and reducing agent were added. The reaction mixture was then evaporated and the product purified by chromatography over silica gel eluting with dichloromethane containing an increasing concentration of ammonium hydroxide/methanol (0-20%) to yield the title compound; ESMS *m*/*z* 446 [M+H]⁺.

### f) 6-[3-(2-tert-Butoxycarbonylamino-ethylamino)-propyl]-1-ethyl-4-oxo-1,4-dihydroquinoline-3-carboxylic acid sodium salt.

A solution of **Intermediate 6e** (0.23 g, 0.52 mmol) in dioxane (7 mL) was treated with 2 M sodium hydroxide (3 mL) and water (2 mL). After stirring at room temperature for 16 h the mixture was treated with solid carbon dioxide, and the residue chromatographed over silica gel eluting with dichloromethane containing an increasing concentration of ammonium hydroxide/methanol (0-20%) to yield the title compound as a white solid; ¹H NMR δ (CD₃OD) 1.39 (9H, s), 1.48(3H, brd. t), 1.93-2.00 (2H, m), 2.86-2.98 (6H, m); 3.28-3.30 (2H, m), 4.46 (2H, brd. q), 7.75-7.80 (2H, m), 8.27 (1H, brd d), 8.89 (1H, s).

### g) 6-[3-(2-Amino-ethylamino)-propyl]-1-ethyl-4-oxo-1,4-dihydro-quinoline-3-carboxylic acid bis-trifluoroacetate.

**Intermediate 6f** (0.2 g, 0.51 mmol) was treated with trifluoroacetic acid (2 mL) After stirring at room temperature for 2 h the mixture was diluted with dichloromethane and evaporated to yield the title compound as a brown gum; ESMS *m*/*z* 318 [M+H]⁺.

### Intermediate 7: 4"-O-(1-Imidazolyl-carbonyl)-6-O-methyl-erythromycin A

6-*O*-Methyl-erythromycin A (30 g, 40.1 mmol) in tetrahydrofuran (100 mL) was treated portionwise with carbonyldiimidazole (16 g, 97 mmol) with ice bath cooling. After 1 h the cooling bath was removed. After a futher 48 h, tetrahydrofuran (100 mL) and water (200 mL) were added causing slow precipitation of the title compound, which was collected by filtration and dried to give the title compound (24.7 g). Extraction of the mother liquors with ether gave a further 8.5 g of material which was precipitated from tetrahydrofuran solution with water to give a further portion of the title compound (3.92 g, total 28.64 g); ESMS *m*/*z* 842 [M+H]⁺.

### Intermediate 8: 2'-O-Acetyl-4"-O-(1-imidazolyl-carbonyl)-azithromycin

To a solution of 2'-*O*-Ac-azithromycin (5 g) in dry toluene (75 mL), 2 mL of Et₃N and 1.12g of 1,1'-carbonyldiimidazole were added. The reaction mixture was stirred at room temperature for 24 hours and then another portion of 1.12g of 1,1'-carbonyldiimidazole was added. The reaction mixture was then stirred at room temperature for another 24 hours. The reaction mixture was washed with saturated aqueous NaHCO₃ (2x35 mL) and the aqueous layer was extracted with toluene (2x20 mL). The combined organic layers were dried over K₂CO₃ and evaporated *in vacuo* to give the title compound (5.6 g). ESMS m/z 885 (MH+).

### Intermediate 9: 6-[(2-Amino-ethylamino)ethyl]-1-ethyl-4-oxo-1,4-dihydro-quinolone-3-carboxylic acid ethyl ester

### a) 6-((E)-2-Carbamoyl-vinyl)-1-ethyl-4-oxo-1,4-dihydro-quinolone-3-carboxylic acid ethyl ester.

A solution of 1-ethyl-6-iodo-4-oxo-1,4-dihydro-quinolone-3-carboxylic acid ethyl ester (1.0 g, 2.7 mmol), sodium bicarbonate and acrylamidet ( 0.22 g, 3.05 mmol) in DMF (5 mL) and water (1.5 mL) was degassed for 0.5 h. Palladium acetate ( 0.017 g, 0.076 mmol) was added and the resultant mixture was heated at 90°C. After 16 h the mixture was cooled and diluted with water, the title compound precipitated as a cream coloured solid. ESMS *m*/*z* 315 (MH⁺).
t Cancer suspect agent.

### b) 6-((E)-2-Carbamoyl-vinyl)-1-ethyl-4-oxo-1,4-dihydro-quinolone-3-carboxylic acid ethyl ester.

A solution of **Intermediate 9a** (0.39g, 1.24 mmol) was hydrogenated over 10% palladium on charcoal at 1 atmosphere and ambient temperature. After 24 h the catalyst was removed by filtration and the filtrate evaporated to yield the title compound as a pale yellow powder. ESMS *m*/*z* 317 (MH⁺).

### c) 6-(2-Ethyloxycarbonylamino-ethyl)-1-ethyl-4-oxo-1,4-dihydro-quinolin-3-carboxylic acid ethyl ester.

A solution of **Intermediate 9b** (0.2 g, 0.63 mmol) and mercuric acetate (0.24 g, 0.75 mmol) in DMF (6 mL) and ethanol (1.2 mL, 20 mmol) was treated with NBS (0.15 g, 0.83 mmol). After stirring at room temperature for 16 h the mixture was diluted with water and the organic material extracted with ethyl acetate (3 x 25 mL). The combined organic extracts were dried and evaporated. The crude product was chromatographed over silica gel eluting with dichloromethane containing an increasing concentration of methanol. (0 to 5%) to yield the title compound as a yellow solid. ESMS *m*/*z* 361 (MH⁺).

### d) 6-(Amino-ethyl)-1-ethyl-4-oxo-1,4-dihydroquinoline-3-carboxylic acid ethyl ester hydroiodide salt.

To a solution of **Intermediate 9c** (0.19 g, 0.53 mmol) in dichloromethane (4 mL) was added iodotrimethylsilane (0.76 mL). After stirring at room temperature for 24 h the liquid phase was decanted and the residual solid was dissolved in ethanol to hydrolyse the remaining iodotrimethylsilane. The ethanol was evaporated and the residue washed with dichloromethane to yield the title compound as a brown gum. ESMS *m*/*z* 289 (MH⁺).

### e) 6-(2-(t-Butoxycal-bonylamino-ethylamino)ethyl)-1-ethyl-4-oxo-1,4-dihydroquinolin-3-carboxylic acid ethyl ester.

Using a procedure identical to that described for the preparation of **Intermediate 6e** a mixture of **Intermediate 9d** (0.219 g, 0.53 mmol) and *t*-butyl (2-oxoethyl)carbamate (0.1 g, 0.64 mmol) gave the title compound as beige solid ESMS *m*/*z* 432 (MH⁺).

### f) 6-(2-(t-Butoxycarbonylamino-ethylamino)ethyl)-1-ethyl-4-oxo-1,4-dihydroquinoline-3-carboxylic acid sodium salt.

Using a procedure identical to that described for the preparation of **Intermediate 6f** a mixture of **Intermediate 9e** (0.067 g, 0.16 mmol) gave the title compound as white solid ESMS *m*/*z* 404 (MH⁺).

### g) 6-[(2-Amino-ethylamino)ethyl-1-ethyl-4-oxo-1,4-dihydro-quinoline-3-carboxylic acid trifluoroacetate salt.

Using a procedure identical to that described for the preparation of **Intermediate 6g** a mixture of **Intermediate 9f** (0.05 g, 0.12 mmol) gave the title compound as white solid ESMS *m*/*z* 303 (MH⁺).

### Intermediate 10: 6-[3-(2-Amino-ethoxy)-propyl]-1-ethyl-4-oxo-1,4-dihydro-quinoline-3-carboxylic acid formate salt

### a) 6-[3-(2-Butoxycarbonylamino-ethoxy)-prop-1-ynyl]-1-ethyl-4-oxo-1,4-dihydroquinoline-3-carboxylic acid ethyl ester

Using a procedure identical to that described for the preparation of **Intermediate 6b** a mixture of **Intermediate 6a** (1.81 g, 3.19 mmol) and *t*-butyl (2-prop-2-ynyloxy-ethyl)carbamic acid (1.08 g, 5.4 mmol) gave the title compound as yellow solid ESMS *m*/*z* 443 (MH⁺).

### b) 6-[3-(2-Butoxycarbonylamino-ethoxy)-propyl]-1-ethyl-4-oxo-1,4-dihydroquinoline-3-carboxylic acid ethyl ester.

Using a procedure identical to that described for the preparation of **Intermediate 6c** Intermediate 10a (0.592 g, 1.34 mmol) gave the title compound as yellow solid ESMS *m*/*z* 447 (MH⁺).

### c) 6-[3-(2-Butoxycarbonytamino-ethoxy)-propyl]-1-ethyl-4-oxo-1,4-dihydroquinoline-3-carboxylic acid sodium salt.

Using a procedure identical to that described for the preparation of **Intermediate 6f Intermediate 10b** (0.481 g, 1.08 mmol) gave the title compound as yellow solid ESMS *m*/*z* 419 (MH⁺).

### d) 6-[3-(2-Amino-ethoxy)-propyl]-1-ethyl-4-oxo-1,4-dihydro-quinoline-3-carboxylic acid trifluoroacetate salt.

Using a procedure identical to that described for the preparation of **Intermediate 6g Intermediate 10c** (0.451 g, 1.08 mmol) gave the title compound as yellow solid. ESMS *m*/*z* 319 (MH⁺).

### Intermediate 11: 6-{[2-amino-ethylamino]methyl}-1-ethyl-4-oxo-1,4-dihydroquinolin-3-carboxylic acid ditrifluoroacetate salt

### a) 6-(Amino-methyl)-1-ethyl-4-oxo-1,4-dihydroquinoline-3-carboxylic acid ethyl ester.

A mixture of 6-cyano-1-ethyl-4-oxo-1,4-dihydroquinolin-3-carboxylic ethyl ester (prepared according to GB 830832), and 5% rhodium on alumina suspended in tetrahydrofuran (75 mL) and 2M ammonia in methanol (25 mL) was hydrogenated at 1 atmosphere and ambient temperature. After 72 h the catalyst was removed by filtration and the resultant filtrate evaporated to dryness. The residual bright yellow gum was treated with dichloromethane and re-evaporated to yield a bright yellow powder. ESMS *m*/*z* 275 (MH⁺).

### b) 6-(2-t-Butoxycarbonylamino-ethylamino)methyl)-1-ethyl-4-oxo-1,4-dihydroquinoline-3-carboxylic acid ethyl ester.

Using a procedure identical to that described for the preparation of Intermediate 6e a mixture of **Intermediate 11a** (0.155 g, 0.57 mmol) and and *t*-butyl (2-oxoethyl)carbamate (0.11 g, 0.68 mmol) gave the title compound as beige solid ESMS *m*/*z* 418 (MH⁺).

### c) 6-(2-t-Butoxycarbonylamino-ethylamino)methyl)-1-ethyl-4-oxo-1,4-dihydroquinoline-3-carboxylic acid sodium salt.

Using a procedure identical to that described for the preparation of **Intermediate 6f** a mixture of **Intermediate 11b** (0.131 g, 0.31mmol) gave the title compound as white solid ESMS *m*/*z* 412 (MNa⁺).

### d) 2-(Amino-ethylamino)methyl)-1-ethyl-4-oxo-1,4-dihydroquinoline-3-carboxylic acid bis-trifluoroacetate salt.

Using a procedure identical to that described for the preparation of **Intermediate 6g** a mixture of **Intermediate 11c** (0.090g, 0.23 mmol) gave the title compound as white solid ESMS *m*/*z* 426 (MH⁺).

### Intermediate 12: 6-(2-Amino-ethylsulfanyl)-1-ethyl-4-oxo-1,4-dihydro-quinoline-3-carboxylic acid trifluoroacetate salt

### a) 6-Bromo-1-ethyl-4-oxo-1,4-dihydroquinoline-3-carboxylic acid ethyl ester.

A mixture of potassium carbonate (2.95 g, 21.2 mmol) and 6-bromoquinolone-3-carboxylic acid in dimethylformamide (25 mL) was heated to 40°C under argon for 10 minutes and iodoethane (3.4 mL, 42.4 mmol) was added. After 14 h the mixture was cooled and the DMF evaporated. The residue was treated with water (40 mL), cooled to 5°C and filtered under vacuum. The resultant cream-coloured solid was dried under vacuum to yield the title compound; ¹H NMR δ [(CD₃)₂SO] 1.41 (3H, t, J = 7.1 Hz), 1.54 (3H, J = 7.2 Hz), 4.24 (2H, q, J = 7.2 Hz), 4.40 (2H, q, J = 7.1 Hz), 7.34 (1H, d, J = 9 Hz), 7.76 (1H, dd, J = 2.4 & 9 Hz), 8.65 (1H, d, J = 2.4 Hz), 8.49 (1H, s).

### b) 6-(2-t-Butoxycarbonylaminoethylsulfanyl)-1-ethyl-4-oxo-1,4-dihydro-quinolone-3-carboxylic acid ethyl ester.

A mixture of *N*-Boc-cysteinamine (0.35 g, 2 mmol), **Intermediate 12a** (0.32 g, 1 mmol) and potassium carbonate (0.28 g, 2 mmol) was heated in DMSO (10 mL) at 90°C. After 16 h the mixture was cooled and partitioned between water and dichloromethane, the organic layer separated, dried and evaporated to yield the crude product. Chromatography over silica gel eluting with dichloromethane containing an increasing concentration of methanol/ammonium hydroxide the title compound was obtained as a white solid; ESMS *m*/*z* 421 (MH⁺).

### c) 6-(2-Amino-ethylsulfanyl)-1-ethyl-4-oxo-1,4-dihydro-quinoline-3-carboxylic acid ethyl ester trifluoroacetate salt.

Using a procedure identical to that described for the preparation of **Intermediate 6f** a mixture of **Intermediate 12b** 0.068 g, 0.17 mmol) gave the title compound as a pale yellow gum. ESMS *m*/*z* 321 (MH⁺).

### d) 6-[2-(2-t-Butoxycarbonylamino-ethylamino)-ethylsulfanyl]-1-ethyl-4-oxo-1,4-dihydro-quinoline-3-carboxylic acid ethyl ester formate salt.

Using a procedure identical to that described for the preparation of **Intermediate 6e** a mixture of **Intermediate 12c** ( 1.07 g, 2.5 mmol) and and *t*-butyl (2-oxoethyl)carbmate (0.47 g, 2.9 mmol) gave the title compound, after MDAP purification eluting with 0.5% formic acid in water and 0.5% formic acid in actonitrile, as white solid ESMS *m*/*z* 464 (MH⁺).

### e) 6-[2-(2-t-Butoxycarbonylamino-ethylamino)-ethylsulfanyl]-1-ethyl-4-oxo-1,4-dihydro-quinoline-3-carboxylic acid.

Using a procedure identical to that described for the preparation of **Intermediate 6f** a mixture of **Intermediate 12d** ( 0.5 g, 1.07 mmol) gave the title compound as a pale yellow solid ESMS *m*/*z* 436 (MH⁺).

### f) 6-[2-(2-Amino-ethylamino)-ethylsulfanyl]-1-ethyl-4-oxo-1,4-dihydro-quinoline-3-carboxylic acid trifluoroacetic acid salt.

Using a procedure identical to that described for the preparation of **Intermediate 6g** a mixture of **Intermediate 12e** (0.043 g, 0.1 mmol) gave the title compound as a pale yellow solid. ESMS *m*/*z* 336 (MH⁺).

### Intermediate 13: 6-{2-[(2-Amino-ethyl)-methyl-amino]-ethylsulfanyl}-1-ethyl-4-oxo-4-dihydro-puinoline-3-carboxylic acid

### a) 6-{2-[(2-Butoxycarbonylamino-ethyl)-methyl-amino]-ethylsulfanyl}-1-ethyl-4-oxo-1,4-dihydro-quinoline-3-carboxylic acid ethyl ester.

A solution of **Intermediate 12d** (0.36 g, 0.62 mmol) in DMF (1 mL) was treated with iodomethane (0.043 mL, 0.069 mmol) and potassium carbonate (0.13 g, 0.93 mmol) at room temperature. After stirring for 50 h the solvent was evaporated and the residue chromatographed over silica gel eluting with dichloromethane containing an increasing concentration of 10% ammonium hydroxide/methanol (0 to 10%) to yield the title compound as a colourless oil. ESMS *m*/*z* 478 (MH⁺).

### b) 6-{2-[(2-Butoxycarbonylamino-ethyl)-methyl-amino]-ethylsulfanyl}-1-ethyl-4-oxo-1,4-dihydro-quinoline-3-carboxylic acid sodium salt.

Using a procedure identical to that described for the preparation of **Intermediate 6f** a mixture of **Intermediate 13a** (0.132 g, 0.28 mmol) gave the title compound as a pale yellow solid ESMS *mlz* 450 (MH⁺).

### c) 6-{2-[(2-Amino-ethyl)-methyl-amino]-ethylsulfanyl}-1-ethyl-4-oxo-1,4-dihydroquinoline-3-carboxylic acid di-trifluoroacetate salt.

Using a procedure identical to that described for the preparation of **Intermediate 6g Intermediate 13b** ( 0.125 g, 0.27 mmol) gave the title compound as colourless gum. ESMS *m*/*z* 350 (MH⁺).

### Intermediate 14

### 7-Chloro-1-cyclonropyl-6-(2-hydroxy-ethylamino)-4-oxo-1,4-dihydro-quinoline-3-carboxylic acid (A) and 1-Cyclopropyl-6-fluoro-7-(2-hydroxy-ethylamino)-4-oxo-1,4-dihydro-quinoline-3-carboxylic acid (B)

To a solution of ethanolamine (55.5 mL) in N-methyl pyrrolidinone (500 mL) at 95 °C, 7-chloro-1-cyclopropyl-6-fluoro-4-oxo-1,4-dihydro-quinoline-3-carboxylic acid (50.0 g) was slowly added under vigorous stirring. The temperature was increased to 105 °C and the reaction mixture was stirred at this temperature for 22 hours. The reaction mixture was cooled to about 60 °C and poured into MeOH (800 mL). This mixture was stirred in an ice bath and the precipitate was filtered off and dried affording a mixture of **Intermediate 14A** and **Intermediate 14B** (49 g ) in a 1:1 ratio.
**Intermediate 14A:** MS; m/z (ES): 322.99 [MH]⁺
**Intermediate 14B:** MS; m/z (ES): 307.02 [MH]⁺

### Intermediate 15

### 7-Chloro-6-[2-(2-cyano-ethoxy)-ethylamino]-1-cyclopropyl-4-oxo-1,4-dihydroquinoline-3-carboxylic acid (A) and 7-[2-(2-Cyano-ethoxy)-ethylamino]-1-cyclopropyl-6-fluoro-4-oxo-1,4-dihydroquinoline-3-carboxylic acid (B)

A solution of a mixture of **Intermediate 14A** and **Intermediate 14B** (14 g) in acrylonitrile (140 mL) and DBU (14 mL) was stirred at 70 °C for 16 hours. The solvent was evaporated and the residue dissolved in i-PrOH (50 mL). Water (50 mL) was added and the pH value adjusted to 4. The precipitate was filtered and then triturated with methanol. After filtration, 5.35 g of pure **Intermediate 15A** was obtained. The mother liquor was left overnight at 4 °C and 4.4 g of **Intermediate 15B** precipitated.
**Intermediate 15A**: ¹H-NMR (500 MHz, DMSO-d6) δ: 8.56 (s, 1H), 8.23 (s, 1H), 7.40 (s, 1H), 5.93 (t, NH), 3.83 (qv, 1H), 3.72 (t, 2H), 3.67 (t, 2H), 3.46 (q, 2H), 2.79 (t, 2H), 1.30 (q, 2H), 1.18 (q, 2H). ¹³C-NMR (75 MHz, DMSO-d6) δ: 176.52, 166.09, 145.72, 142.72, 132.17, 126.37, 125.38, 119.15, 118.99, 106.14, 102.76, 67.93, 65.05, 42.40, 35.77, 18.01, 7.32. MS; m/z (ES): 376.02 [MH]⁺
**Intermediate 15B:** ¹H-NMR (500 MHz, DMSO-d6) δ: 8.55 (s, 1H), 7.76 (d, 1H), 7.22 (d, 1H), 3.74 (t, 2H+1H), 3.67 (t, 2H), 3.52 (q, 2H), 2.78 (t, 2H), 1.31 (m, 2H), 1.18 (m, 2H). ¹³C-NMR (75 MHz, DMSO-d6) δ: 175.80, 166.20, 148.12, 146.89, 142.55, 140.30, 119.22, 108.79, 106.10, 96.68, 68.29, 65.17, 42.06, 35.70, 17.99, 7.48. MS; m/z (ES): 360.04 [MH]⁺

### Intermediate 16: 6-[2-(3-Amino-propoxy)-ethylamino]-7-chloro-1-cyclopropyl-4-oxo-1,4-dihydro-quinoline-3-carboxylic acid (A) and

### 6-[2-(3-Amino-propoxy)-ethylamino]-1-cyclopropyl-4-oxo-1,4-dihydro-quinoline-3-carboxylic acid (B)

A high pressure reactor was filled with a mixture of **Intermediate 15A** (1 g), 120 mL of acetic acid and 0.340 g of PtO₂ at a H₂ pressure of 4.6 bar for 24 hours. The reaction mixture was then filtered through celite and the acetic acid was evaporated in vacuo. Crude product was precipitated from CH₂Cl₂-diisopropyl ether yielding 0.59g of the title compounds. LC-MS showed that major product is **Intermediate 16B** (1.3:1).

### Intermediate 17: 2'-O-Acetyl-11-O-methyl-4"-O-(1-imidazolyl-carbonyl)-azithromycin

Starting from 2'-OAc-11-O-methyl azithromycin and 1,1'-carbonyldiimidazole and according to procedure of **Intermediate 8** the title compound was prepared. MS m/z 899.4 (MH⁺).

### Example 1: 4"-O-3-[2-(3-Carboxy-7-chloro-1-cyclopropyl-4-oxo-1,4-dihydro-quinolin-6-yloxy)-ethoxy]-propylcarbamoyl}- azithromycin (A) and

### 4"-O-{3-[2-(3-Carboxy-1-cyclopropyl-4-oxo-1,4-dihydro-quinolin-6-yloxy)-ethoxy]-propylcarbamoyl}-azithromycin (B)

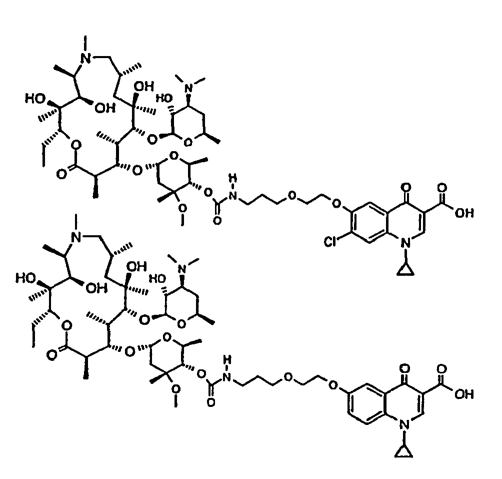

To a DMF (12 mL) solution of **Intermediates 3A and 3B** (0.55 g), a solution of **Intermediate 8** (2.3 g) in DMF (5 mL) was added. DBU (0.6 mL) was then added and the reaction mixture was stirred at room temperature for 20 hours and then at 30 °C for another 3 hours. The reaction mixture was diluted with water (80 mL) and extracted with EtOAc (4 x 30 mL). Organic layer was dried over K₂CO₃ and evaporated *in vacuo.* 40 mL of MeOH was added and the reaction mixture was stirred at 50 °C for 24 hours. Evaporation of MeOH yielded the product which was precipitated twice from EtOAc:n-hexane yielding 1g of the title product. LC-MS showed that the product is (2:1) for **1A:1B**;MS; m/z (ES): 1154.29 [MH]⁺, 1120.34 [MH]⁺.

### Example 2: 4"-O-{3-[2-(3-Carboxy-1-cyclopropyl-4-oxo-1,4-dihydro-quinolin-6-yloxy)-ethoxy]-propylcarbamoyl}-azithromycin

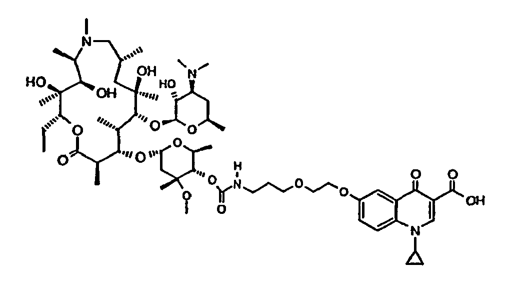

A high pressure reactor was filled with a mixture of 0.5 g **Example 1A and 1B**, 50 mL MeOH and 0.25 g 10% Pd/C at a H₂ pressure of 8 bar for 24 hours. The catalyst was filtered off and the solvent evaporated *in vacuo.* 40 mL of aqueous NaHCO₃ and 30 mL of CH₂Cl₂ was added and the pH was adjusted to 9. The mixture was extracted twice with CH₂Cl₂. The combined organic layers were dried over K₂CO₃ and evaporated *in vacuo.* The product was precipitated from EtOAc:n-hexane yielding 0.345 g of **Example 2**; ¹³C-NMR (300 MHz, CDCl₃) δ: 178.98, 178.90, 156.46, 146.78, 135.73, 125.26, 124.68, 106.51, 102.55, 94.76, 83.04, 79.19, 77.87, 77.03, 74.27, 73.82, 73.63, 73.18, 70.94, 70.11, 69.15, 69.05, 67.98, 67.86, 65.52, 63.33, 62.46. 49.58, 45.18, 42-30, 42.07, 40.33, 38.67, 36.28, 35.11, 34.26, 30.12, 28.79, 27.55, 26.79, 21.98, 21.74, 21.26, 21.09, 17.85, 16.22, 14.64, 11.24, 9.11, 8.22, 7.39. MS; m/z (ES): 1120.42 [MH]⁺ .

### Example 3: 4"-O-{2-[2-(3-Carboxy-1-ethyl-4-oxo-1,4-dihydro-auinolin-6-yloxy)-ethylamino]-ethylcarbamoyl}-6-O-methyl-erythromycin A monoformate salt

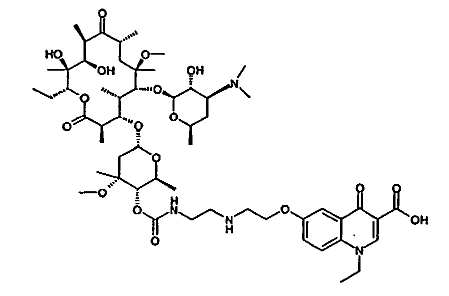

**Intermediate 5** (0.025 g, 0.045 mmol) and **Intermediate 7** (0.038 g, 0.045 mmol) in THF (4 mL) were treated with DBU (0.03 mL, 0.2 mmol). The mixture was heated at 50°C for 70 h then evaporated to dryness. The residue was purified by preparative reverse phase HPLC (MeCN/H₂O/0.1%HCO₂H eluent) to give the title compound; ESMS *m*/*z* 1093 [M+H]⁺.

### Example 4: 4"-O-{2-[3-(3-Carboxy-1-ethyl-4-oxo-1,4-dihydro-quinolin-6-yl)-propylamino]-ethylcarbamoyl}-6-O-methyl-erythromycin A

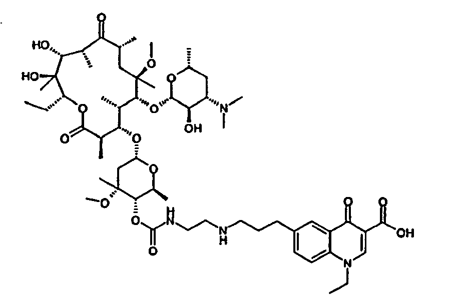

A mixture of **Intermediate 7** (0.20 g, 0.24 mmol) and **Intermediate 6** (0.112 g, 0.21 mmol) in DMF (2 mL) was added DBU (0.15 mL, 0.1 mmol). After stirring at room temperature for 96 h the mixture was concentrated and chromatographed over silica gel eluting with dichloromethane containing an increasing concentration of methanol/ammonium hydroxide (0-30%) to yield the title compound as white solid; ESMS *m*/*z* 1091 (MNH₄⁺).

### Example 5: 4"-O-{2-[3-(3-Carboxy-1-ethyl-4-oxo-1,4-dihydro-quinolin-6-yl)-methylpropylamino]-ethylcarbamoyl}-6-O-methyl-erythromycin A

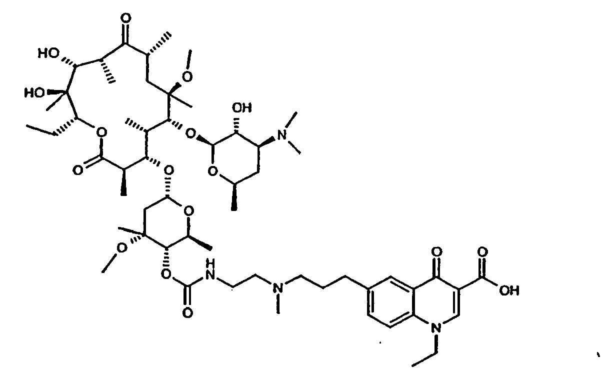

A solution of **Example 4** (0.072 g, 0.066 mmol) in chloroform (0.9 mL), aqueous formaldehyde (3.6 µL, 0.132 mmol) and formic acid (9.2 µL, 0.24 mmol) was heated at 60°C. After 85 mins the mixture was cooled, concentrated and the residue purified by chromatography over silica gel eluting with dichloromethane containing an increasing concentration of methanol/ammonium hydroxide (0-10%) to yield 26% of the title compound as a white solid; ESMS *m*/*z* 1106 (MH⁺).

### Example 6: 4"-O-{2-[2-(3-Carboxy-1-ethyl-4-oxo-1,4-dihydro-quinolin-6-yl)-ethylamino]-ethylcarbamoyl}-6-O-methyl-erythromycin A

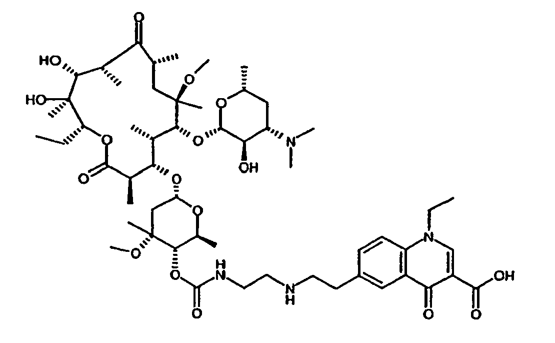

A mixture of **Intermediate 7** (0.134 g, 0.16 mmol) and **Intermediate 9** (0.085g, 0.16 mmol) in DMF (2 mL) was added DBU (0.15 mL, 0.1 mmol). After stirring at room temperature for 48 h the mixture was concentrated and chromatographed over silica gel eluting with dichloromethane containing an increasing concentration of methanol/ammonium hydroxide (0-30%) to yield the title compound as pale yellow solid; ESMS *m*/*z* 1078 (MH⁺).

### Example 7: 4"-O-{2-[3-(3-Carboxy-1-ethyl-4-oxo-1,4-dihydro-quinolin-6-yl)-propyloxy]-ethylcarbamoyl}-6-O-methyl-erythromycin A

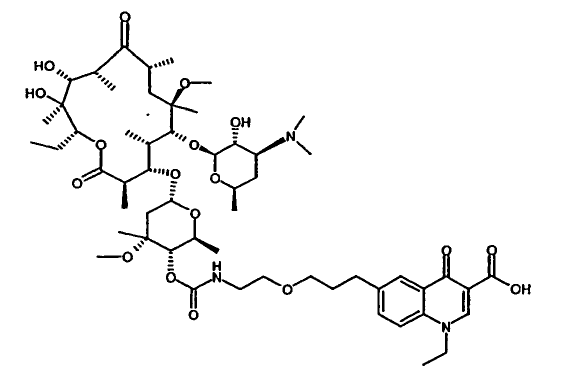

A mixture of **Intermediate 7** (1.0 g, 1.2 mmol) and **Intermediate 10** (0.258 mg, 0.6 mmol) in DMF (20 mL) was added DBU (0.9 mL, 6 mmol). After stirring at room temperature for 48 h the mixture was concentrated and chromatographed over silica gel eluting with dichloromethane containing an increasing concentration of methanol/ammonium hydroxide (0-5%) to yield the title compound as pale yellow solid; ESMS *m*/*z* 1093 (MH⁺).

### Example 8: 4"-O-{[2-(3-Carboxy-1-ethyl-4-oxo-1,4-dihydro-quinolin-6-yl)-methylamino]-ethylcarbamoyl}-6-O-methyl-erythromycin A

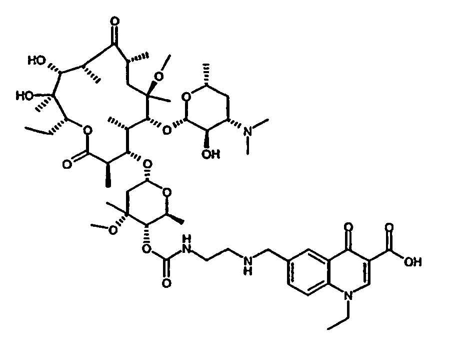

A mixture of **Intermediate** 7 (0.23 g, 0.27 mmol) and **Intermediate 11** (0.082 mg, 0.15 mmol) in DMF (3 mL) was added DBU (0.2 mL, 0.13 mmol). After stirring at room temperature for 48 h the mixture was concentrated and chromatographed over silica gel eluting with dichloromethane containing an increasing concentration of methanol/ammonium hydroxide (0-30%) to yield the title compound as white solid; ESMS *m*/*z* 1064 (MH⁺).

### Example 9: 4"-O-{[3-(3-Carboxy-1-ethyl-4-oxo-1,4-dihydro-6-quinolinylsulfanyl)-ethylamino]-ethylcarbamoyl}-6-O-methyl-erythromycin A

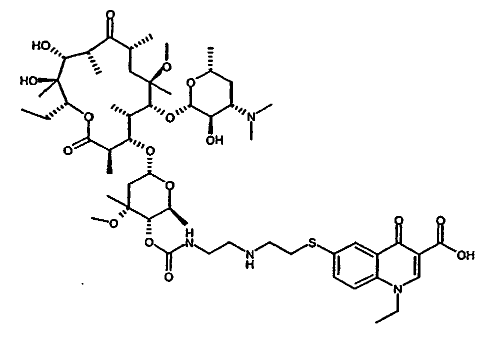

A mixture of **Intermediate 7** (0.10 g, 0.12 mmol) and **Intermediate 12** (0.057 mg, 0.1 mmol) in DMF (3 mL) was added DBU (0.2 mL, 0.13 mmol). After stirring at room temperature for 20 h the mixture was concentrated and chromatographed over silica gel eluting with dichloromethane containing an increasing concentration of methanol/ammonium hydroxide (0-10%) to yield the title compound as white solid; ESMS *m*/*z* 1110 (MH⁺).

### Example 10: 4"-O-{[3-(3-Carboxy-1-ethyl-4-oxo-1,4-dihydro-6-quinolinylsulfanyl)-ethylamino]-ethylcarbamoyl}-6-O-methyl-erythromycin A

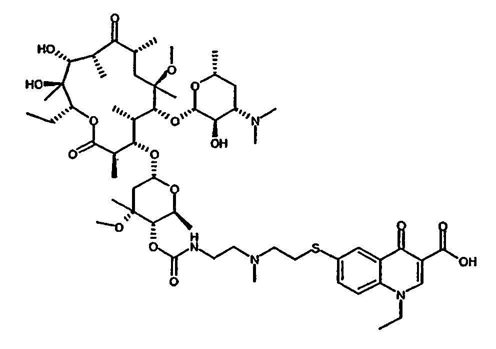

A mixture of **Intermediate 7** (0.08 g, 0.1 mmol) and **Intermediate 13** (0.042 mg, 0.072 mmol) in DMF (2 mL) was added DBU (0.2 mL, 0.13 mmol). After stirring at room temperature for 24 h the mixture was concentrated and purified by MDAP. The resultant formate salt was converted to the title compound by chromatography over silica gel eluting with dichloromethane containing an increasing concentration of methanol/ammonium hydroxide (0-20%) to yield a white solid; ESMS *m*/*z* 1123 (MH⁺).

### Example 11: 4"-O-{3-[2-(3-Carboxy-7-chloro-1-cyclopropyl-4-oxo-1,4-dihydro-quinolin-6-yloxy)-ethoxy]-propylcarbamoyl}-azithromycin (A) and 4"-O-{3-[2-(3-Carboxy-1-cyclopropyl-4-oxo-1,4-dihydro-dihydro-quinolin-6-yloxy)-ethoxy]-propylcarbamoyl}-azithromycin (B)

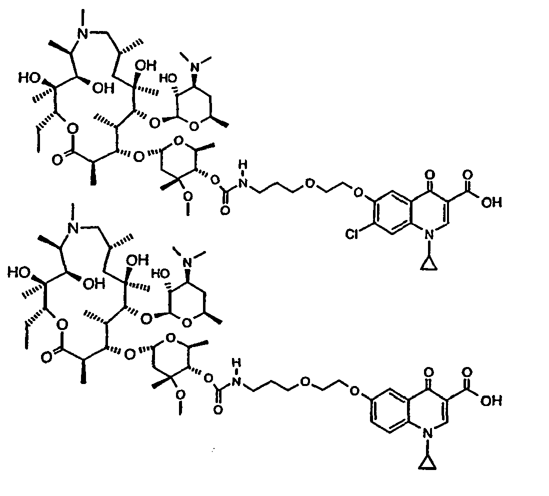

Intermediate 8 (2.3 g) in DMF (5 mL) was added to a DMF (12 mL) solution of **Intermediate 16A** and **Intermediate 16B** (0.55 g). DBU (0.6 mL) was then added and the reaction mixture was stirred at room temperature for 20 hours and then at 30°C for another 3 hours. The reaction mixture was diluted with water (80 mL) and extracted with EtOAc (4 x 30 mL). The organic layer was dried over K₂CO₃ and evaporated *in vacuo.* 40 mL of MeOH was added and the reaction mixture was stirred at 50 °C for 24 hours. Evaporation of MeOH yielded the product which was precipitated twice from EtOAc:n-hexane yielding 1 g of the title product. LC-MS showed that major product was **Example 11A** (2:1).
MS; m/z (ES): 1154.29 [MH]⁺, 1120.34 [MH]⁺

### Example 12: 4"-O-{3-[2-(3-Carboxy-1-cyclopropyl-4-oxo-1,4-dihydro-quinolin-6-yloxy)-ethoxy]-propylcarbamoyl}-azithromycin

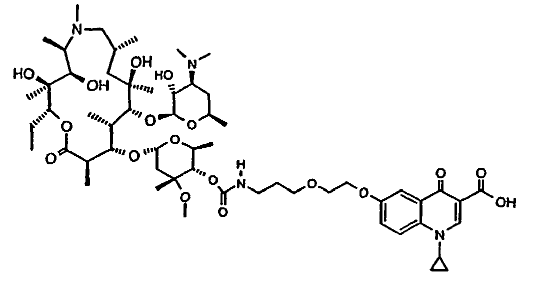

A high pressure reactor was filled with a mixture of 0.5 g of **Example 11A** and **Example 11B**, 50 mL MeOH and 0.25 g 10% Pd/C at a H₂ pressure of 8 bar for 24 hours. The catalyst was then filtered off and the solvent evaporated *in vacuo.* 40 mL of aqueous NaHCO₃ and 30 mL of CH₂Cl₂ was added and the pH was adjusted to 9. The mixture was then extracted twice with CH₂Cl₂. The combined organic layers were dried over K₂CO₃ and evaporated *in vacuo.* The product was precipitated from EtOAc:n-hexane yielding 0.345 g of the title compound.
¹³C-NMR (300 MHz, CDCl₃) δ: 178.98, 178.90, 156.46, 146.78, 135.73, 125.26, 124.68, 106.51, 102.55, 94.76, 83.04, 79.19, 77.87, 77.03, 74.27, 73.82, 73.63, 73.18, 70.94, 70.11, 69.15, 69.05, 67.98, 67.86, 65.52, 63.33, 62.46, 49.58, 45.18, 42.30, 42.07, 40.33, 38.67, 36.28, 35.11, 34.26, 30.12, 28.79, 27.55, 26.79, 21.98, 21.74, 21.26, 21.09, 17.85, 16.22, 14.64, 11.24, 9.11, 8.22, 7.39.
MS; m/z (ES): 1120.42 [MH]⁺.

### Example 13: 4"-O-{3-[2-(3-Carboxy-7-chloro-1-cyclopropyl-4-oxo-1,4-dihydro-quinolin-6-ylamino)-ethoxy]-propylcarbamoyl}-azithromycin (A) and 4"-O-{3-[2-(3-Carboxy-1-cyclopropyl-4-oxo-1,4-dihydro-quinolin-6-ylamino)-ethoxy]-propylcarbamoyl}-azithromycin (B)

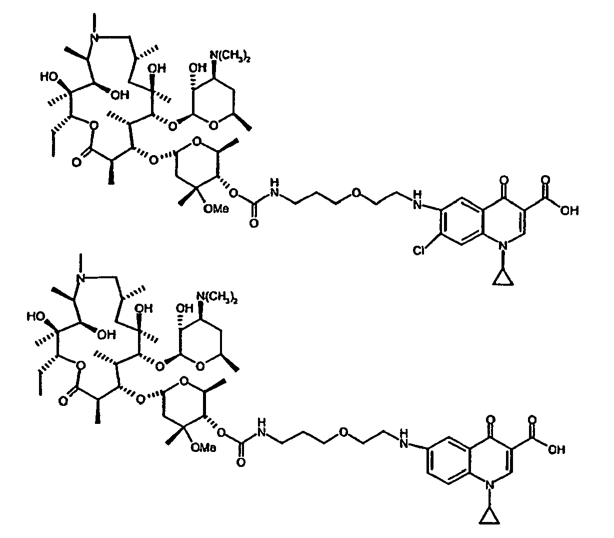

**Intermediate 8** (2.3 g) in DMF (5 mL) and DBU were added to a DMF (12 ml) solution of **Intermediate 16A** and **Intermediate 16B** (0.5 g). The reaction mixture was stirred at room temperature for 20 hours and then at 35 °C for another hour. The reaction mixture was diluted with water (200 mL) and extracted with EtOAc (5 x 40 mL). The organic layer was dried over K₂CO₃ and evaporated *in vacuo.* 50 mL of MeOH was then added and the reaction mixture was stirred at 60 °C for 24 hours. The MeOH was evaporated and the crude product was precipitated twice from EtOAc:n-hexane and then purified by column chromatography (eluent CH₂Cl₂:MeOH:NH₃ = 90:13:2) yielding 0.350g of the title compounds. LC-MS showed that favourable product is **Example 13B** (1.7:1). **Example 13(A)** [MS+2H⁺]⁺² 577.76, **Example 13(B)** [MS+2H⁺]⁺² = 560.76.

### Example 14: 4"-O-{3-[2-(3-Carboxy-1-cyclopropyl-4-oxo-1,4-dihydro-quinolin-6-ylamino)-ethoxy]-propylcarbamoyl}-azithromycin

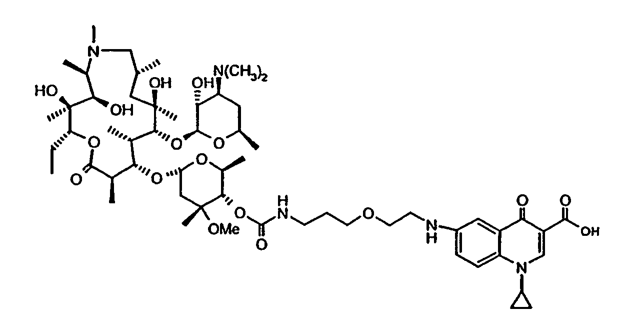

A high pressure reactor was filled with a mixture of 0.34 g of **Example 13A** and **Example 13B**, 40 mL MeOH and 0.170 g 10% Pd/C at a H₂ pressure of 6 bar for 24 hours. The catalyst was filtered off and the solvent evaporated *in vacuo.* 40 ml of aqueous NaHCO₃ and 30 ml of CH₂Cl₂ were added and the pH was adjusted to 9. The aqueous layer was extracted with CH₂Cl₂ (2 x 30 mL). The combined organic layers were dried over K₂CO₃ and evaporated *in vacuo.* The product was precipitated from EtOAc.n-hexane yielding 0.247 g of the title compound (according to LC-MS 95.2% pure product). [MS+2H⁺]⁺² 561.43.

### Example 15: 4"-O-{3-[2-(3-Carboxy-7-chloro-1-cyclopropyl-4-oxo-1,4-dihydro-quinolin-6-yloxy)-ethoxy]-propylcarbamoyl}-clarithromycin (A) and 4"-O-{3-[2-(3-Carboxy-1-cuclopropyl-4-oxo-1,4-dihydro-quinolin-6-yloxy)-ethoxy]-propylcarbamoyl}-clarithromycin (B)

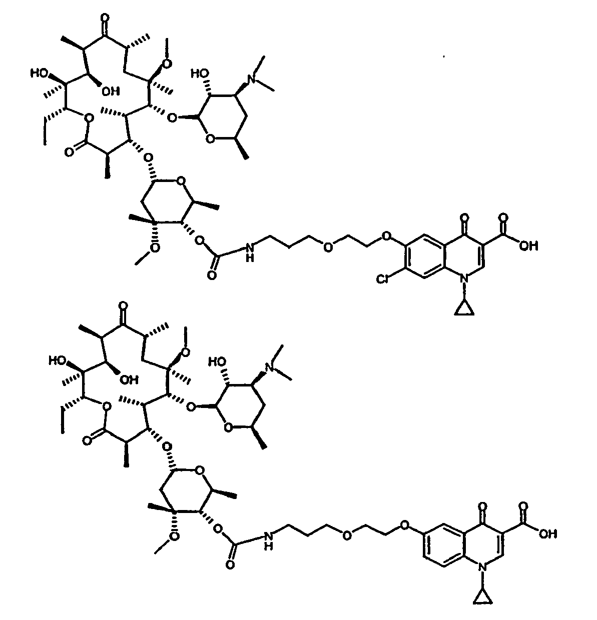

A solution of **Intermediate 7** (2.7 g, 3 mmol, 2 eq.) in DMF (12 mL) was added to a DMF (10 mL) solution of **Intermediate 3A** and **Intermediate 3B** (0.58 g, 1.5 mmol). DBU (0.7 mL, 4.5 mmol, 3 eq.) was then added and the reaction mixture was stirred at room temperature for 20 hours. The reaction mixture was diluted with water (80 mL) and extracted with EtOAc (4 x 35 mL). The organic layer was dried over K₂CO₃ and evaporated *in vacuo.* 40 mL of MeOH was added and the reaction mixture was stirred at 50 °C for 24 hours. Evaporation of MeOH yielded the product which was precipitated from CH₂Cl₂: diisopropyl-ether yielding 0.7 g of the title compounds. LC-MS showed that the favorable product is **Example 15A** (1.5:1).

MS; m/z (ES): 1154.8 [MH]⁺, 1120.8 [MH]⁺.

### Example 16: 4"-O-{3-[2-(3-Carboxy-1-cyclopropyl-4-oxo-1.4-dihydro-quinolin-6-yloxy)-ethoxy]-propylcarbamoyl}-clarithromycin

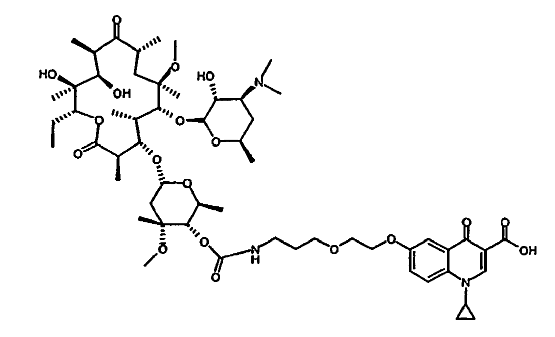

A high pressure reactor was filled with a mixture of 0.5 g of **Example 15A** and **Example 15B**, 40 mL MeOH and 0.25 g 10% Pd/C by pressure of H₂ at 8 bar for 24 hours. The catalyst was filtered and the solvent evaporated /*n vacuo.* 40 mL of aqueous NaHCO₃ and 15 mL of CH₂Cl₂ were added and the pH was adjusted to 9. The aqueous layer was extracted twice with CH₂Cl₂. The combined organic layers were dried over K₂CO₃ and evaporated *in vacuo.* The product was precipitated from EtOAc:n-hexane yielding 0.22 g of the title compound.
MS; m/z (ES): 1120.8 [MH]+

### Example 17: 4"-O-{3-[2-(3-Carboxy-7-chloro-1-cyclopropyl-4-oxo-1,4-dihydro-quinolin-6-yloxy)-ethoxy]-propylcarbamoyl}-11-O-methylazithromycin (A) and 4"-O-{3-[2-(3-Carboxy-1-cyclopropyl-4-oxo-1,4-dihydro-quinolin-6-yloxy)-ethoxy]-propylcarbamoyl}-11-O-methylazithromycin (B)

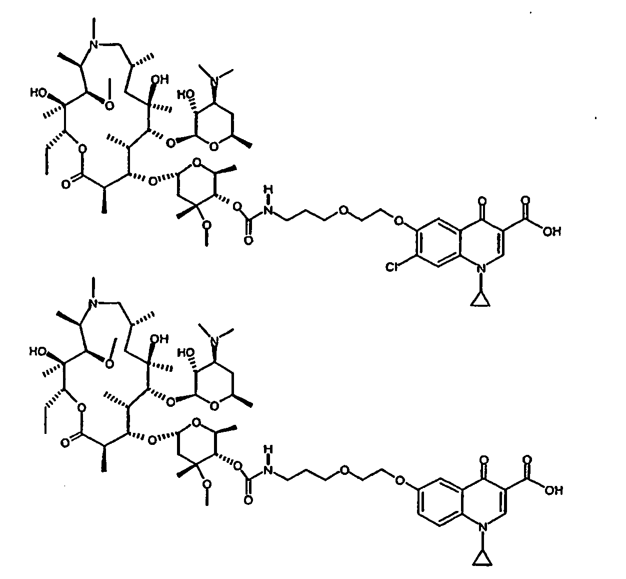

**Intermediate 17** (1.67 g) dissolved in 5 mL DMF was added to a suspension of **Intermediate 3A** and **Intermediate 3B** (0.5 g) in 12 mL DMF. 0.43 mL of DBU was then added and the reaction mixture was stirred at room temperature overnight. 40 ml of water was added and the mixture was extracted with ethyl acetate (3x15 mL). The combined organic layers were dried over Na₂SO₄ and evaporated *in vacuo*. 30 ml of MeOH was added and the reaction mixture was stirred at 60 °C for 8 hours. The MeOH was evaporated *in vacuo,* CH₂Cl₂ was added and the mixture was extracted with brine (5x20 mL). The organic layer was dried over Na₂SO₄ and evaporated. The crude product was precipitated from EtOAc-diisopropyl ether yielding 0.450g of product.
MS; m/z (ES): 569.38 [MH]⁺ (35.46 %), 586.39 [MH]⁺ (59.74 %).

### Example 18: 4"-O-{3-[2-(3-Carboxy-7-chloro-1-cyclopropyl-4-oxo-1,4-dihydro-quinolin-6-ylamino)-ethoxy]-propylcarbamoyl}-11-O-methylazithromycin (A) and 4"-O-{3-[2-(3-Carboxy-1-cyclopropyl-4-oxo-1,4-dihydro-quinolin-6-yloxy)-ethoxy]-propylcarbamoyl}-11-O-methylazithromycin (B)

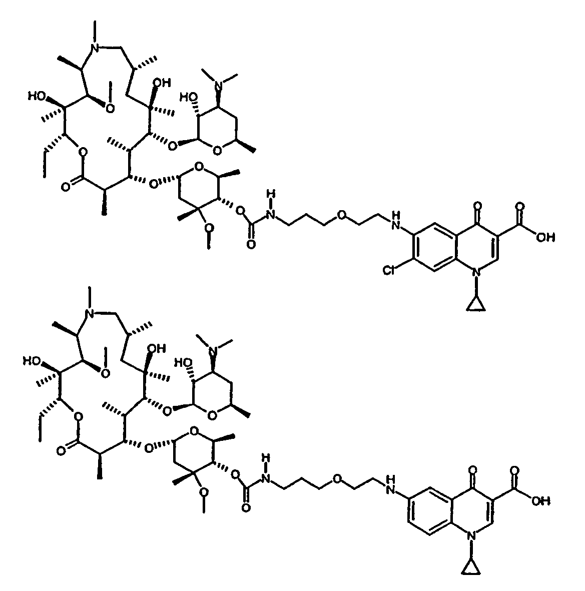

**Intermediate 17** dissolved in 12 mL DMF was added to a suspension of **Intermediate 16A** and **Intermediate 16B** (1 g) dissolved in 25 ml DMF. 1.12 ml of DBU was then added and the reaction mixture was stirred at room temperature overnight. 40 mL of water was added and the mixture was extracted with ethyl acetate (3x15 ml). The combined organic layers were dried over Na₂SO₄ and evaporated *in vacuo.* 30 mL of MeOH was added and the reaction mixture was stirred at 60 °C for 8 hours. The MeOH was evaporated *in vacuo,* CH₂Cl₂ was added and the mixture was extracted with brine (5x20 mL). The organic layer was dried over Na₂SO₄ and evaporated. The crude product was precipitated from EtOAc-diisopropyl ether yielding 1.543 g of product.
MS; m/z (ES): 569.38 [MH]⁺ (35.46 %). 586.39 [MH]⁺ (59.74 %).

### Example 19: 4"-O-{3-[2-(3-Carboxy-1-cyclopropyl-4-oxo-1,4-dihydro-quinolin-6-yloxy)-ethoxy]-propylcarbamoyl}-11-O-methylazithromycin

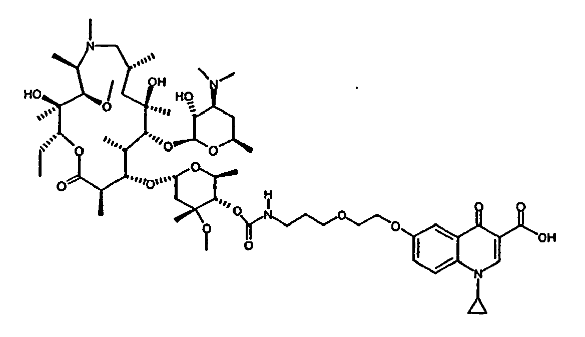

A high pressure reactor was filled with a mixture of 0.3 g of **Example 17A** and **Example 17B,** 50 mL MeOH and 0.150 g 10% Pd/C at a H₂ pressure of 8 bar for 24 hours. The catalyst was filtered off and the solvent evaporated *in vacuo.* 40 mL of aqueous NaHCO₃ and 30 mL of CH₂Cl₂ was added and the pH was adjusted to 9. The mixture was extracted twice with CH₂Cl₂ and the combined organic layers were dried over K₂CO₃ and evaporated *in vacuo.* The product was precipitated from EtOAc:n-hexane yielding 0.150 g of the title compound.
MS; m/z (ES): 1135.41 [MH]⁺ (100 %).

### Example 20: 4"-O-{3-[2-(3-Carboxy-1-cydopropyl-4-oxo-1,4-dihydro-quinolin-6-ylamino)-ethoxy]-propylcarbamoyl}-11-O-methylazithromycin

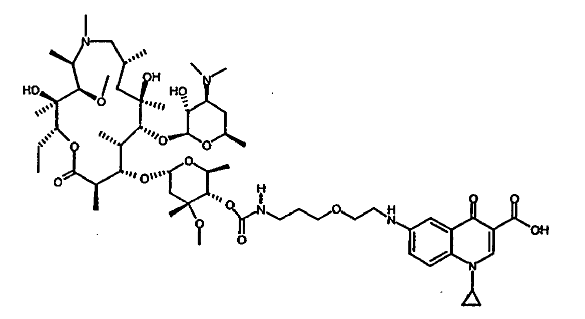

A high pressure reactor was filled with a mixture of 0.461 g of **Example 18A** and **Example 18B**, 60 mL MeOH and 0.230 g 10% Pd/C at a H₂ pressure of 8 bar for 24 hours. The catalyst was filtered off and the solvent evaporated *in vacuo.* 40 mL of aqueous NaHCO₃ and 30 mL of CH₂Cl₂ was added and the pH was adjusted to 9. The mixture was extracted twice with CH₂Cl₂ and the combined organic layers were dried over K₂CO₃ and evaporated *in vacuo.* The product was precipitated from EtOAc:n-hexane yielding 0.220 g of the title compound.
MS; m/z (ES): 1134.43 [MH]⁺ (100 %).

### Biological Data

Using a standard broth dilution method in microtitre, compounds were tested for antibacterial activity. The compounds in the above examples gave minimum inhibitory concentrations (MICs) less than 1 microgram per millilitre against erythromycin-sensitive and erythromycin-resistant strains of *Streptococcus pneumoniae* and *Streptococcus pyogenes.*

In addition, the MIC (µg/mL) of test compounds against various organisms was determined including:
*S. aureus* Smith ATCC 13709, *S. pneumoniae* SP030, *S. pyogenes* 3565, *E. faecalis* ATCC 29212, *H. influenzae* ATCC 49247, *M. catarrhalis* ATCC 23246.

Examples 1-3 have an MIC ≤1 µg/mL against *S. aureus* Smith ATCC 13709, *S*. *pneumoniae* SP030, *S. pyogenes* 3565 and *E. faecalis* ATCC 29212.

Example 1 has an MIC ≤2 µg/mL against *H. influenzae* ATCC 49247 and *M. catarrhalis* ATCC 23246.

Examples 1 and 2 have an MIC ≤0.25 µg/mL against erythromycin resistant strains of *Streptococcus pneumoniae* and *Streptococcus pyogenes.*

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the following claims:

## Claims

1. A compound of formula (I) 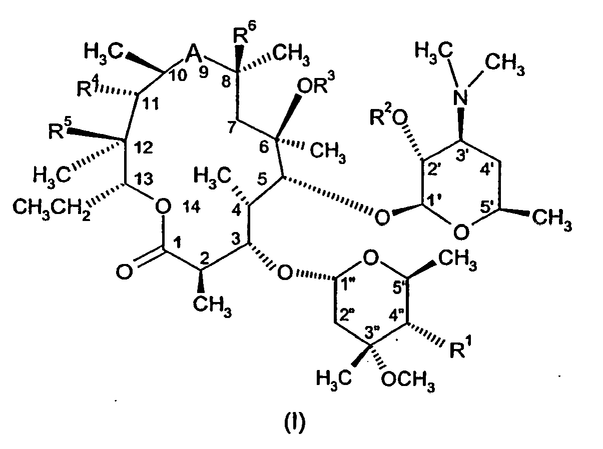 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)N(R¹¹)(CH₂)_{d}XR¹²;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹³,
R⁵ is hydroxy, or
R⁴ and R⁵ taken together with the intervening atoms form a cyclic group having the following structure: 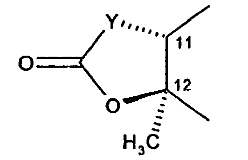 wherein Y is a bivalent radical selected from -CH₂-, -CH(CN)-, -O-, -N(R¹⁴)- and-CH(SR¹⁴)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁵R¹⁶ or - C(O)R¹⁵, or
R⁸ and R⁹ together form =CH(CR¹⁵R¹⁶)_{f}aryl, =CH(CR¹⁵R¹⁶)_{f}heterocyclyl, =CR¹⁵R¹⁶ or =C(R¹⁵)C(O)OR¹⁵, wherein the alkyl, aryl and heterocyclyl groups are optionally substituted by up to three groups independently selected from R¹⁷;
R¹⁰ is -OR¹⁸, C₁₋₆alkyl, -(CH₂)_{g}aryl, -(CH₂)_{g}heterocyclyl or -(CH₂)ₕO(CH₂)ᵢOR⁷, wherein each R¹⁰ group is optionally substituted by up to three groups independently selected from R¹⁷;
R¹¹ is hydrogen or C₁₋₆alkyl;
R¹² is a heterocyclic group having the following structure: 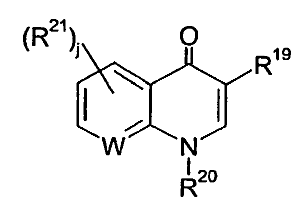 or 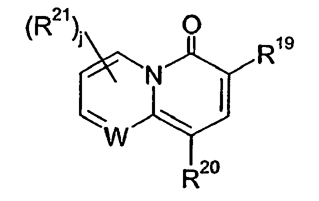 R¹³ is hydrogen or C₁₋₆alkyl;
R¹⁴ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹⁵ and R¹⁶ are each independently hydrogen or C₁₋₆alkyl;
R¹⁷ is halogen, cyano, nitro, trifluoromethyl, azido, -C(O)R²², -C(O)OR²², -OC(O)R²²,-OC(O)OR²², -NR²³C(O)R²⁴, -C(O)NR²³R²⁴, -NR²³R²⁴, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁵R¹⁶, halogen and -OR¹⁵, and the aryl and heteroaryl groups are optionally substituted by up to five groups independently selected from halogen, cyano, nitro, trifluoromethyl, azido,-C(O)R²⁵, -C(O)OR²⁵, -OC(O)OR²⁵, -NR²⁶C(O)R²⁷, -C(O)NR²⁶R²⁷, -NR²⁶R²⁷, hydroxy, C₁₋₆alkyl and C₁₋₆alkoxy;
R¹⁸ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁸, -S(O)ₙR²⁸, -NR²⁸R²⁹, -CONR²⁸R²⁹, halogen and cyano;
R¹⁹ is hydrogen, -C(O)OR³⁰, -C(O)NHR³⁰, -C(O)CH₂NO₂ or -C(O)CH₂SO₂R⁷;
R²⁰ is hydrogen, C₁₋₄alkyl optionally substituted by hydroxy or C₁₋₄alkoxy, C₃₋₇cycloalkyl, or optionally substituted phenyl or benzyl;
R²¹ is halogen, C₁₋₄alkyl, C₁₋₄thioalkyl, C₁₋₄alkoxy, -NH₂, -NH(C₁₋₄alkyl) or -N(C₁₋₄alkyl)₂;
R²² is hydrogen, C₁₋₁₀alkyl, -(CH₂)ₚaryl or -(CH₂)ₚheteroaryl;
R²³ and R²⁴ are each independently hydrogen, -OR¹⁵, C₁₋₆alkyl, -(CH₂)_{q}aryl or-(CH₂)_{q}heterocyclyl;
R²⁵ is hydrogen, C₁₋₁₀alkyl, -(CH₂)ᵣaryl or -(CH₂)ᵣheteroaryl;
R²⁶ and R²⁷ are each independently hydrogen, -OR¹⁵, C₁₋₆alkyl, -(CH₂)ₛaryl or-(CH₂)ₛheterocydyl;
R²⁸ and R²⁹ are each independently hydrogen, C₁₋₄alkyl or C₁₋₄alkoxyC₁₋₄alkyl;
R³⁰ is hydrogen,
C₁₋₆alkyl optionally substituted by up to three groups independently selected from halogen, cyano, C₁₋₄alkoxy optionally substituted by phenyl or C₁₋₄alkoxy,-C(O)C₁₋₆alkyl, -C(O)OC₁₋₆alkyl, -OC(O)C₁₋₆alkyl, -OC(O)OC₁₋₆alkyl,-C(O)NR³³R³⁴, -NR³³R³⁴ and phenyl optionally substituted by nitro or -C(O)OC₁₋₆alkyl,
-(CH₂)_{w}C₃₋₇cycloalkyl,
-(CH₂)_{w}heterocyclyl,
-(CH₂)_{w}heteroaryl,
-(CH₂)_{w}aryl,
C₃₋₆alkenyl, or
C₃₋₆alkynyl;
R³¹ is hydrogen, C₁₋₄alkyl, C₃₋₇cycloalkyl, optionally substituted phenyl or benzyl, acetyl or benzoyl;
R³² is hydrogen or R²¹, or R³² and R²⁰ are linked to form the bivalent radical -O(CH₂)₂- or -(C^{H}2)ₜ-;
R³³ and R³⁴ are each independently hydrogen or C₁₋₆alkyl optionally substituted by phenyl or -C(O)OC₁₋₆alkyl, or
R³³ and R³⁴, together with the nitrogen atom to which they are bound, form a 5 or 6 membered heterocyclic group optionally containing one additional heteroatom selected from oxygen, nitrogen and sulfur,
X is -U(CH₂)ᵥB-, -U(CH₂)ᵥ- or a group selected from: 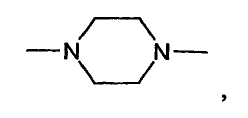 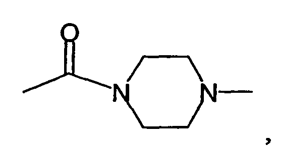 and 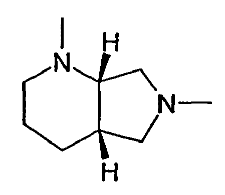 U and B are independently a divalent radical selected from -N(R³¹)-, -O-, -S(O)_{z}-,-N(R³¹)C(O)-, -C(O)N(R³¹)- and -N[C(O)R³¹]-;
W is -C(R³²)- or a nitrogen atom;
d is an integer from 2 to 5;
e is an integer from 2 to 4;
f, g, h, m, p, q, r, s and w are each independently integers from 0 to 4;
i is an integer from 1 to 6;
j, k, n and z are each independently integers from 0 to 2;
t is 2 or 3;
v is an integer from 1 to 8;
or a pharmaceutically acceptable derivative thereof.

2. A compound according to claim 1 wherein A is -C(O)- or -N(R⁷)-CH₂-

3. A compound according to claim 1 or claim 2 wherein X is -U(CH₂)ᵥB- or -U(CH₂)ᵥ.

4. A compound according to any one of the preceding claims wherein d is 2 or 3.

5. A compound according to any one of the preceding claims wherein R¹² is a heterocyclic group of the following formula: 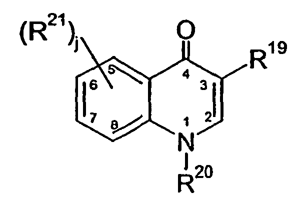 wherein the heterocyclic is linked in the 6 or 7 position and j, R¹⁹, R²⁰ and R²¹ are as defined in claim 1.

6. A compound according to claim 1 as defined in any one of Examples 1 to 20, or a pharmaceutically acceptable derivative thereof.

7. A compound selected from:
4"-O-3-[2-(3-Carboxy-7-chloro-1-cyclopropyl-4-oxo-1,4-dihydro-quinolin-6-yloxy)-ethoxy]-propylcarbamoyloxy}- azithromycin;
4"-O-{3-[2-(3-Carboxy-1-cyclopropyl-4-oxo-1,4-dihydro-quinolin-6-yloxy)-ethoxy]-propylcarbamoyloxy}-azithromycin;
4"-O-{2-[3-(3-carboxy-1-ethyl-4-oxo-1,4-dihydro-quinolin-6-yl)-propyloxy]-ethylcarbamoyl}-6-O-methyl-erythromycin A;
4"-*O*-{3-[2-(3-carboxy-1-cyclopropyl-4-oxo-1,4-dihydro-quinolin-6-ylamino)-ethoxy]-propylcarbamoyl}-azithromycin;
4"-*O*-{3-[2-(3-carboxy-1-cyclopropyl-4-oxo-1,4-dihydro-quinolin-6-yloxy)-ethoxy]-propylcarbamoyl}-11-*O*-methylazithromycin; and
4"-*O*-{3-[2-(3-carboxy-1-cydopropyl-4-oxo-1,4-dihydro-quinolin-6-ylamino)-ethoxy]-propylcarbamoyl}-11-*O*-methylazithromycin;
or a pharmaceutically acceptable derivative thereof.

8. A process for the preparation of a compound as claimed in claim 1 which comprises:
a) reacting a compound of formula (II) 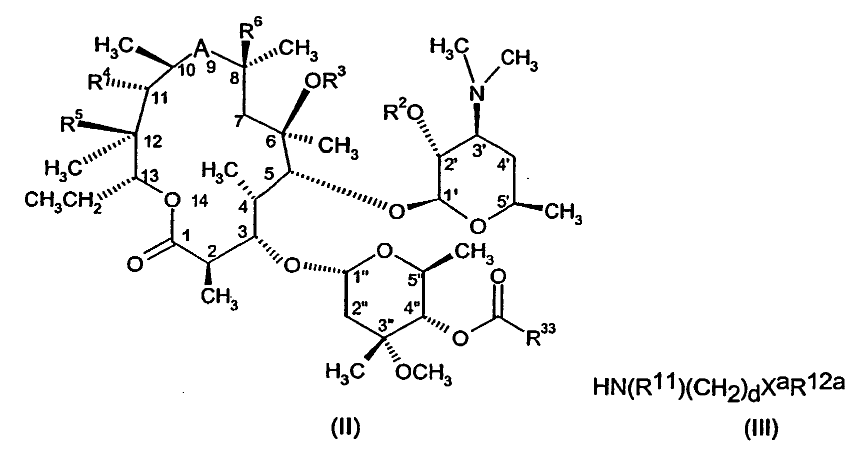 with a suitable amine (III), wherein R³³ is an activating group, and X^{a} and R^{12a} are X and R¹² as defined in claim 1 or groups convertible to X and R¹²;
b) reacting a compound of formula (IV) 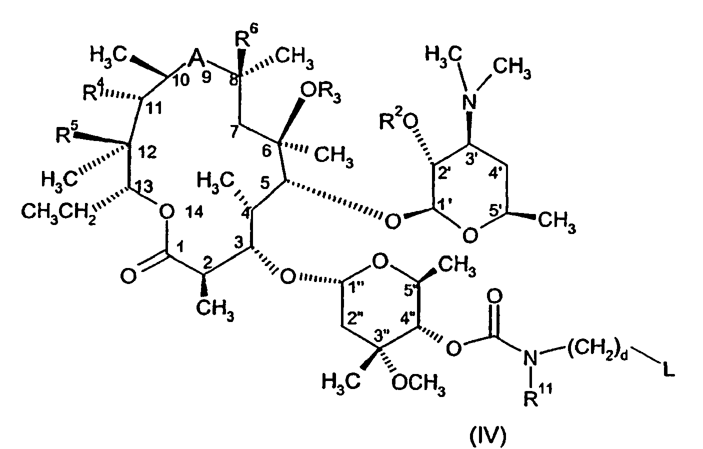 with a compound of formula X^{a}R^{12a} (V), wherein R^{12a} is R¹² as defined in claim 1 or a group convertible to R¹² and X^{a} is -U(CH₂)ᵥ- or -U(CH₂)ᵥB-, or a group convertible to-U(CH₂)ᵥ- or -U(CH₂)_{V}B-, in which U is a group selected from -N(R³¹)- and -S-, and L is suitable leaving group, to produce a compound of formula (I) wherein U is a group selected from -N(R³¹)- and -S-; or
c) converting one compound of formula (I) into another compound of formula (I),
and thereafter, if required, subjecting the resulting compound to one or more of the following operations:
i) removal of the protecting group R²,
ii) conversion of X^{a}R^{12a} to XR¹², and
iii) conversion of the resultant compound of formula (I) into a pharmaceutically acceptable derivative thereof.

9. A compound as claimed in any one of claims 1 to 7 for use in therapy.

10. The use of a compound as claimed in any one of claims 1 to 7 in the manufacture of a medicament for use in the treatment or prophylaxis of systemic or topical microbial infections in a human or animal body.

11. A pharmaceutical composition comprising at least one compound as claimed in any one of claims 1 to 7 in association with a pharmaceutically acceptable excipient, diluent and/or carrier.

12. A compound of formula (IA) 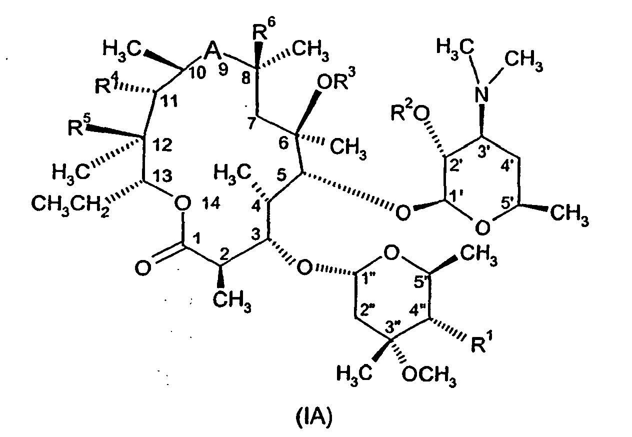 wherein
A is a bivalent radical selected from -C(O)-, -C(O)NH-, -NHC(O)-, -N(R⁷)-CH₂-, -CH₂-N(R⁷)-, -CH(NR⁸R⁹)- and -C(=NR¹⁰)-;
R¹ is -OC(O)N(R¹¹)(CH₂)_{d}XR¹²;
R² is hydrogen or a hydroxyl protecting group;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R¹³,
R⁵ is hydroxy, or
R⁴ and R⁵ taken together with the intervening atoms form a cyclic group having the following structure: 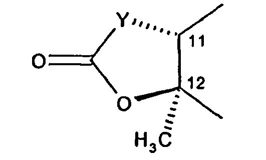 wherein Y is a bivalent radical selected from -CH₂-, -CH(CN)-. -O-, -N(R¹⁴)- and-CH(SR¹⁴)-;
R⁶ is hydrogen or fluorine;
R⁷ is hydrogen or C₁₋₆alkyl;
R⁸ and R⁹ are each independently hydrogen, C₁₋₆alkyl, -C(=NR¹⁰)NR¹⁵R¹⁶ or-C(O)R¹⁵, or
R⁸ and R⁹ together form =CH(CR¹⁵R¹⁶)_{f}aryl, =CH(CR¹⁵R¹⁶)_{f}heterocyclyl, =CR¹⁵R¹⁶ or =C(R¹⁵)C(O)OR¹⁵, wherein the alkyl, aryl and heterocyclyl groups are optionally substituted by up to three groups independently selected from R¹⁷;
R¹⁰ is -OR¹⁸, C₁₋₆alkyl, -(CH₂)_{g}aryl, -(CH₂)_{g}heterocyclyl or -(CH₂)ₕO(CH₂)ᵢOR⁷, wherein each R¹⁰ group is optionally substituted by up to three groups independently selected from R¹⁷;
R¹¹ is hydrogen or C₁₋₆alkyl;
R¹² is a heterocyclic group having the following structure: 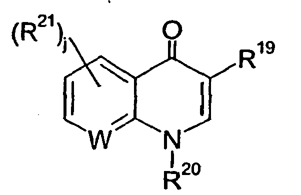 or 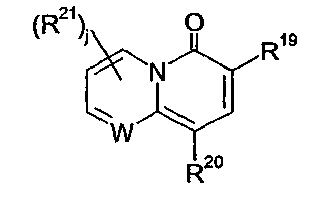 R¹³ is hydrogen or C₁₋₆alkyl;
R¹⁴ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹⁵ and R¹⁶ are each independently hydrogen or C₁₋₆alkyl;
R¹⁷ is halogen, cyano, nitro, trifluoromethyl, azido, -C(O)R²², -C(O)OR²², -OC(O)R²²,-OC(O)OR²², -NR²³C(O)R²⁴, -C(O)NR²³R²⁴, -NR²³R²⁴, hydroxy, C₁₋₆alkyl, -S(O)ₖC₁₋₆alkyl, C₁₋₆alkoxy, -(CH₂)ₘaryl or -(CH₂)ₘheteroaryl, wherein the alkoxy group is optionally substituted by up to three groups independently selected from -NR¹⁵R¹⁶, halogen and -OR¹⁵, and the aryl and heteroaryl groups are optionally substituted by up to five groups independently selected from halogen, cyano, nitro, trifluoromethyl, azido,-C(O)R²⁵, -C(O)OR²⁵, -OC(O)OR²⁵, -NR²⁶C(O)R²⁷, -C(O)NR²⁶R²⁷, -NR²⁶R²⁷, hydroxy, C₁₋₆alkyl and C₁₋₆alkoxy;
R¹⁸ is hydrogen, C₁₋₆alkyl, C₃₋₇cycloalkyl, C₃₋₆alkenyl or a 5 or 6 membered heterocyclic group, wherein the alkyl, cycloalkyl, alkenyl and heterocyclic groups are optionally substituted by up to three substituents independently selected from optionally substituted 5 or 6 membered heterocyclic group, optionally substituted 5 or 6 membered heteroaryl, -OR²⁸, -S(O)ₙR²⁸, -NR²⁸R²⁹, -CONR²⁸R²⁹, halogen and cyano;
R¹⁹ is hydrogen, -C(O)OR³⁰, -C(O)NHR³⁰ or -C(O)CH₂NO₂;
R²⁰ is hydrogen, C₁₋₄alkyl optionally substituted by hydroxy or C₁₋₄alkoxy, C₃₋₇cycloalkyl, or optionally substituted phenyl or benzyl;
R²¹ is halogen, C₁₋₄alkyl, C₁₋₄thioalkyl, C₁₋₄alkoxy, -NH₂, -NH(C₁₋₄alkyl) or -N(C₁₋₄alkyl)₂;
R²² is hydrogen, C₁₋₁₀alkyl, -(CH₂)ₚaryl or -(CH₂)ₚheteroaryl;
R²³ and R²⁴ are each independently hydrogen, -OR¹⁵, C₁₋₆alkyl, -(CH₂)_{q}aryl or-(CH₂)_{q}heterocyclyl;
R²⁵ is hydrogen, C₁₋₁₀alkyl, -(CH₂)ᵣaryl or -(CH₂)ᵣheteroaryl;
R²⁶ and R²⁷ are each independently hydrogen, -OR¹⁵, C₁₋₆alkyl, -(CH₂)ₛaryl or-(CH₂)ₛheterocyclyl;
R²⁸ and R²⁹ are each independently hydrogen, C₁₋₄alkyl or C₁₋₄alkoxyC₁₋₄alkyl;
R³⁰ is hydrogen or C₁₋₆alkyl optionally substituted by up to three groups independently selected from halogen, C₁₋₄alkoxy, -OC(O)C₁₋₆alkyl and -OC(O)OC₁₋₆alkyl;
R³¹ is hydrogen, C₁₋₄alkyl, C₃₋₇cycloalkyl, optionally substituted phenyl or benzyl, acetyl or benzoyl;
R³² is hydrogen or R²¹, or R³² and R²⁰ are linked to form the bivalent radical -O(CH₂)₂- or -(CH2)t-;
X is -U(CH₂)ᵥB-, -U(CH₂)ᵥ- or a group selected from: 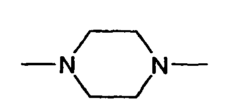 and 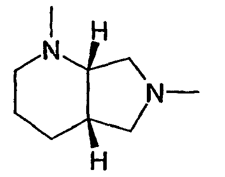 U and B are independently a divalent radical selected from -N(R³¹)-, -O-, -S(O)_{z}-, - N(R³¹)C(O)-, -C(O)N(R³¹)- and -N[C(O)R³¹]-;
W is -C(R³²)- or a nitrogen atom;
d is an integer from 2 to 5;
e is an integer from 2 to 4;
f, g, h, m, p, q, r and s are each independently integers from 0 to 4;
i is an integer from 1 to 6;
j, k, n and z are each independently integers from 0 to 2;
t is 2 or 3;
v is an integer from 2 to 8;
or a pharmaceutically acceptable derivative thereof.

## Patentansprüche

1. Verbindung der Formel (I) 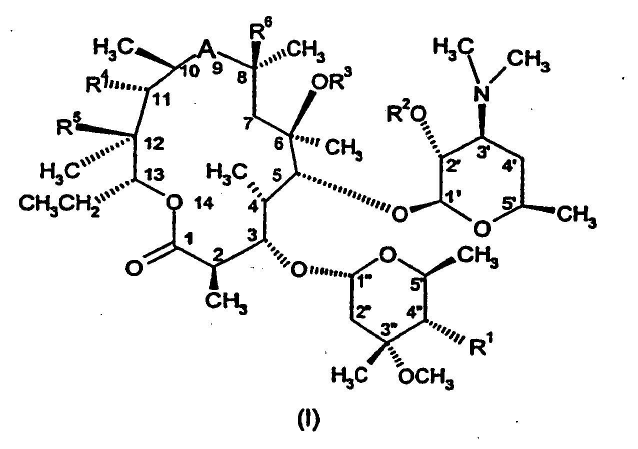 worin bedeuten:
A ist ein bivalenter Rest, ausgewählt aus -C(O)-, -C(O)NH-, -NHC(O)-, -NR7)-CH₂-, -CH₂-N(R⁷)-, -CH(NR⁸R⁹)- und -C(=NR¹⁰)-;
R¹ ist -OC(O)N(R¹¹)(CH₂)_{d}XR¹²;
R² ist Wasserstoff oder eine Hydroxyl-Schutzgruppe;
R³ ist Wasserstoff, C₁₋₄-Alkyl oder C₃₋₆-Alkenyl, gegebenenfalls substituiert durch ein 9- bis 10-gliedriges, anneliertes bicyclisches Heteroaryl;
R⁴ ist Hydroxy, C₃₋₆-Alkenyloxy, gegebenenfalls substituiert durch ein 9- bis 10-gliedriges, anneliertes bicyclisches Heteroaryl, oder C₁₋₆-Alkoxy, gegebenenfalls substituiert durch C₁₋₆-Alkoxy, oder -O(CH₂)ₑNR⁷R¹³;
R⁵ ist Hydroxy oder
R⁴ und R⁵ bilden zusammengenommen mit den dazwischenliegenden Atomen eine cyclische Gruppe, die die folgende Struktur hat: 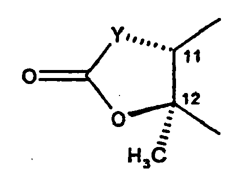 worin Y ein bivalenter Rest, ausgewählt aus -CH₂-, -CH(CN)-, -O-, -N(R¹⁴)- und -CH(SR¹⁴)- ist;
R⁶ ist Wasserstoff oder Fluor;
R⁷ ist Wasserstoff oder C₁₋₆-Alkyl;
R⁸ und R⁹ sind jeweils unabhängig Wasserstoff, C₁₋₆-Alkyl, -C(=NR¹⁰)NR¹⁵R¹⁶ oder -C(O)R¹⁵, oder
R⁸ und R⁹ bilden zusammen =CH(CR¹⁵R¹⁶)_{f}-Aryl, =CH(CR¹⁵R¹⁶)_{f}-Heterocyclyl, =CR¹⁵R¹⁶ oder =C(R¹⁵)C(O)OR¹⁵, worin die Alkyl-, Aryl- und Heterocyclyl-Gruppen gegebenenfalls durch bis zu drei Gruppen, unabhängig aus R¹⁷ ausgewählt, substituiert sind;
R¹⁰ ist -OR¹⁸, C₁₋₆-Alkyl, -(CH₂)_{g}-Aryl, -(CH₂)_{g}-Heterocyclyl oder -(CH₂)ₕO(CH₂)ᵢOR⁷, worin jede R¹⁰-Gruppe gegebenenfalls durch bis zu drei Gruppen, unabhängig aus R¹⁷ ausgewählt, substituiert ist;
R¹¹ ist Wasserstoff oder C₁₋₆-Alkyl;
R¹² ist eine heterocyclische Gruppe, die die folgende Struktur hat: 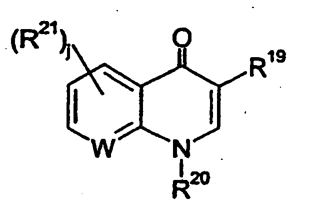 oder 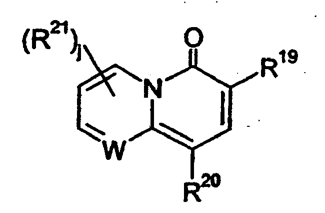
R¹³ ist Wasserstoff oder C₁₋₆-Alkyl;
R¹⁴ ist Wasserstoff oder C₁₋₄-Alkyl, das gegebenenfalls durch eine Gruppe, ausgewählt aus gegebenenfalls substituiertem Phenyl, einem gegebenenfalls substituierten 5- oder 6-gliedrigen Heteroaryl und einem gegebenenfalls substituierten 9- bis 10-gliedrigen annelierten bicyclischen Heteroaryl, substituiert ist;
R¹⁵ und R¹⁶ sind jeweils unabhängig Wasserstoff oder C₁₋₆-Alkyl;
R¹⁷ ist Halogen, Cyano, Nitro, Trifluormethyl, Azido, -C(O)R²², -C(O)OR²², -OC(O)R²², -OC(O)OR²², -NR²²C(O)R²⁴, -C(O)NR²³R²⁴, -NR²³R²⁴, Hydroxy, C₁₋₆-Alkyl, -S(O)ₖ-C₁₋₆-Alkyl, C₁₋₆-Alkoxy, -(CH₂)ₘ-Aryl oder -(CH₂)ₘ-Heteroaryl, worin die Alkoxy-Gruppe gegebenenfalls durch bis zu drei Gruppen substituiert ist, unabhängig ausgewählt aus -NR¹⁵R¹⁶, Halogen und -OR¹⁵ und die Aryl- und Heteroaryl-Gruppen sind gegebenenfalls durch bis zu fünf Gruppen, unabhängig ausgewählt aus Halogen, Cyano, Nitro, Trifluormethyl, Azido, -C(O)R²⁵, -C(O)OR²⁵, -OC(O)OR²⁵, -NR²⁶C(O)R²⁷, -C(O)NR²⁶R²⁷, -NR²⁶R²⁷, Hydroxy, C₁₋₆-Alkyl und C₁₋₆-Alkoxy substituiert;
R¹⁸ ist Wasserstoff, C₁₋₆-Alkyl, C₃₋₇-Cycloalkyl, C₃₋₆-Alkenyl oder eine 5- oder 6-gliedrige heterocyclische Gruppe, worin die Alkyl-, Cycloalkyl-, Alkenyl- und heterocyclischen Gruppen gegebenenfalls durch bis zu drei Substituenten substituiert sind, unabhängig aus einer gegebenenfalls substituierten 5- oder 6-gliedrigen heterocyclischen Gruppe, einem gegebenenfalls substituierten 5- oder 6-gliedrigen Heteroaryl, -OR²⁸, -S(O)NR²⁸, -NR²⁸R²⁹, -CONR²⁸R²⁹, Halogen und Cyano ausgewählt;
R¹⁹ ist Wasserstoff, -C(O)OR³⁰, -C(O)NHR³⁰, -C(O)CH₂NO₂ oder -C(O)CH₂SO₂R⁷;
R²⁰ ist Wasserstoff, C₁₋₄-Alkyl, das gegebenenfalls durch Hydroxy oder C₁₋₄-Alkoxy substituiert ist, C₃₋₇-Cycloalkyl, oder gegebenenfalls substituiertes Phenyl oder Benzyl;
R²¹ ist Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂;
R²² ist Wasserstoff, C₁₋₁₀-Alkyl, -(CH₂)ₚ-Aryl oder -(CH₂)ₚ-Heteroaryl;
R²³ und R²⁴ sind jeweils unabhängig Wasserstoff, -OR¹⁵, C₁₋₆-Alkyl, -(CH₂)_{q}-Aryl oder -(CH₂)_{q}-Heterocyclyl;
R²⁵ ist Wasserstoff, C₁₋₁₀-Alkyl, -(CH₂)ᵣ-Aryl oder -(CH₂)ᵣ-Heteroaryl;
R²⁶ und R²⁷ sind jeweils unabhängig Wasserstoff, -OR¹⁵, C₁₋₆-Alkyl, -(CH₂)ₛ-Aryl oder -(CH₂)-Heterocyclyl;
R²⁸ und R²⁹ sind jeweils unabhängig Wasserstoff, C₁₋₄-Alkyl oder C₁₋₄-Alkoxy-C₁₋₄-Alkyl;
R³⁰ ist Wasserstoff, C₁₋₆-Alkyl, das gegebenenfalls durch bis zu drei Gruppen substituiert ist, unabhängig ausgewählt aus Halogen, Cyano, C₁₋₄-Alkoxy, das gegebenenfalls durch Phenyl oder C₁₋₄-Alkoxy, -C(O)C₁₋₆-Alkyl, -C(O)C₁₋₆-Alkyl, -OC(O)C₁₋₆-Alkyl, -OC(O)OC₁₋₆-Alkyl, -C(O)NR³³R³⁴, -NR³³R³⁴ und Phenyl, gegebenenfalls durch Nitro oder -C(O)OC₁₋₆-Alkyl substituiert, substituiert ist,
-(CH₂)_{w}C₃₋₇-Cycloalkyl,
-(CH₂)_{w}-Heterocyclyl,
-(CH₂)_{w}-Heteroaryl,
-(CH₂)_{w}-Aryl,
C₃₋₆-Alkenyl oder
C₃₋₆-Alkinyl;
R³¹ ist Wasserstoff, C₁₋₄-Alkyl, C₃₋₇-Cycloalkyl, das gegebenenfalls durch Phenyl oder Benzyl substituiert ist, Acetyl oder Benzoyl;
R³² ist Wasserstoff oder R²¹, oder R³² und R²⁰ sind verbunden, um den bivalenten Rest -O(CH₂)₂- oder -(CH₂)ₜ- zu bilden;
R³³ und R³⁴ sind jeweils unabhängig Wasserstoff oder C₁₋₆-Alkyl, das gegebenenfalls durch Phenyl substituiert ist oder -C(O)OC₁₋₆-Alkyl, oder
R³³ und R³⁴ bilden zusammen mit dem Stickstoffatom, an das sie gebunden sind, eine 5- oder 6-gliedrige heterocyclische Gruppe, die gegebenenfalls ein zusätzliches Heteroatom enthält, ausgewählt aus Sauerstoff, Stickstoff und Schwefel;
X ist -U(CH₂)ᵥB-, -U(CH₂)ᵥ- oder eine Gruppe, ausgewählt aus: 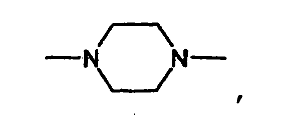 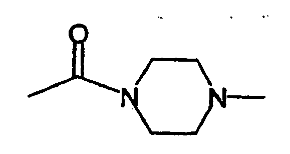 und 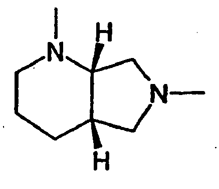
U und B sind unabhängig ein zweiwertiger Rest, ausgewählt aus -N(R³¹)-, -O-, -S(O)_{z}-, -N(R³¹)C(O₎-, -C(O)N(R³¹)- und -N[C(O)R³¹]-;
W ist -C(R³²)- oder ein Stickstoffatom;
d ist eine ganze Zahl von 2 bis 5;
e ist eine ganze Zahl von 2 bis 4;
f, g, h, m, p, q, r, s und w sind jeweils unabhängig ganze Zahlen von 0 bis 4;
i ist eine ganze Zahl von 1 bis 6;
j, k, n und z sind jeweils unabhängig ganze Zahlen von 0 bis 2;
t ist 2 oder 3;
v ist eine ganze Zahl von 1 bis 8;
oder eines pharmazeutisch annehmbaren Derivates davon.

2. Verbindung nach Anspruch 1, worin A gleich -C(O)- oder -N(R⁷)-CH₂- ist.

3. Verbindung nach Anspruch 1 oder Anspruch 2, worin X gleich -U(CH₂)ᵥB- oder -U(CH₂)ᵥ ist.

4. Verbindung nach einem der vorhergehenden Ansprüche, worin d gleich 2 oder 3 ist.

5. Verbindung nach einem der vorhergehenden Ansprüche, worin R¹² eine heterocyclische Gruppe der folgenden Formel ist: 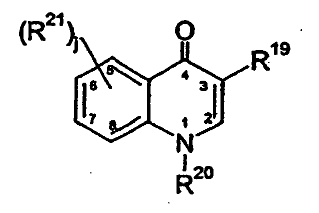 worin der Heterocyclyl in der 6. oder 7. Position gebunden ist und j, R¹⁹, R²⁰ und R²¹ die Bedeutungen haben, wie in Anspruch 1 definiert.

6. Verbindung nach Anspruch 1, wie in einem der Beispiele 1 bis 20 definiert oder deren pharmazeutisch annehmbaren Derivates.

7. Verbindung ausgewählt aus:
4"-O-3-[2-(3-Carboxy-7-chlor-1-cyclopropyl-4-oxo-1,4-dihydro-chinolin-6-yloxy)-ethoxy]-propylcarbamoyloxy)-azithromycin;
4"-O-{3-[2-(3-Carboxy-1-cyclopropyl-4-oxo-1,4-dihydro-chinolin-6-yloxy)-ethoxy]-propylcarbamoyloxy}-azithromycin;
4"-O-{2-[3-(3-Carboxy-1-ethyl-4-oxo-1,4-dihydro-chinolin-6-yl)-propyloxy]-ethylcarbamoyl}-6-O-methyl-erythromycin A;
4"-O-{3-[2-(3-Carboxy-1-cyclopropyl-4-oxo-1,4-dihydro-chinolin-6-ylamino)-ethoxy]-propylcarbamoyl}-azithromycin;
4"-O-{3-[2-(3-Carboxy-1-cyclopropyl-4-oxo-1,4-dihydro-chinolin-6-yloxy)-ethoxy]-propylcarbamoyl}-11-O-methylazithromycin; und
4"-O-{3-[2-(3-Carboxy-1-cyclopropyl-4-oxo-1,4-dihydro-chinolin-6-ylamino)-ethoxy]-propylcarbamoyl}-11-O-methylazithromycin;
oder deren pharmazeutisch annehmbaren Derivates.

8. Verfahren zur Herstellung einer Verbindung nach Anspruch 1, welches umfaßt:
a) Umsetzen einer Verbindung der Formel (II) 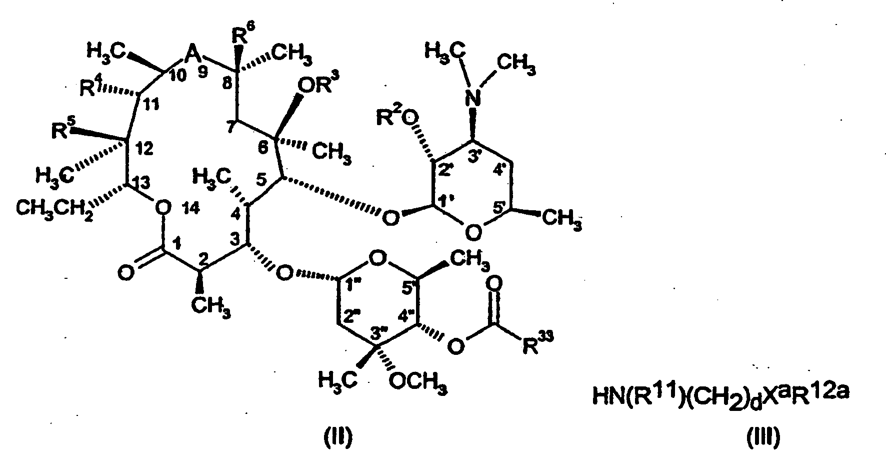 mit einem geeigneten Amin (III), worin R²¹ eine Aktivierungsgruppe ist und X^{a} und R^{12a} sind X und R¹², wie in Anspruch 1 definiert, oder Gruppen, die in X und R¹² konvertierbar sind;
b) Umsetzen einer Verbindung der Formel (IV) 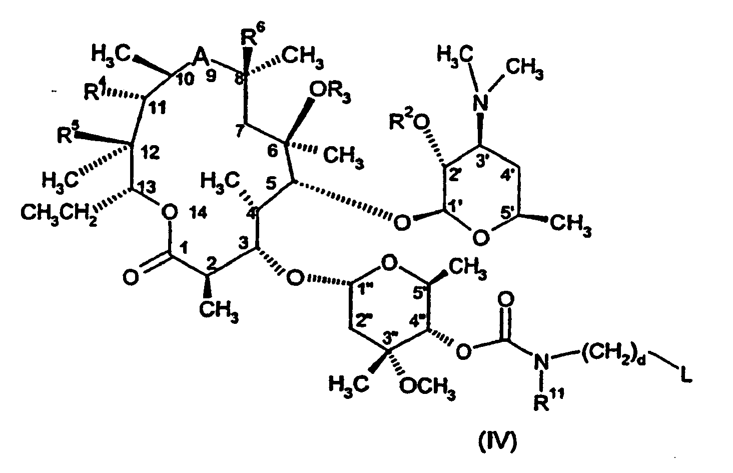 mit einer Verbindung der Formel X^{a}R^{12a} (V), worin R^{12a} gleich R¹², wie in Anspruch 1 definiert, ist oder eine Gruppe, die in R¹² konvertierbar ist und X^{a} ist -U(CH₂)ᵥ- oder -U(CH₂)ᵥB-, oder eine Gruppe, die in -U(CH₂)ᵥ- oder -U(CH₂)ᵥB- konvertierbar ist, in welcher U eine Gruppe ist, ausgewählt aus -N(R³¹)- und -S-, und L ist eine geeignete Abgangsgruppe, um eine Verbindung der Formel (I), worin U eine Gruppe ist, die aus -N(R³¹)- und -S- ausgewählt ist, herzustellen; oder
c) Konvertieren einer Verbindung der Formel (I) in eine andere Verbindung der Formel (I),
und danach, falls erforderlich, Unterwerfen der erhaltenen Verbindung einer oder mehrerer der folgenden Maßnahmen:
i) Entfernen der Schutzgruppe R²,
ii) Konversion von X^{a}R^{12a} in XR¹², und
iii) Konversion der erhaltenen Verbindung der Formel (I) in deren pharmazeutisch annehmbares Derivat.

9. Verbindung nach einem der Ansprüche 1 bis 7, zur Verwendung in der Therapie.

10. Verwendung einer Verbindung, nach einem der Ansprüche 1 bis 7, zur Herstellung eines Medikaments zur Verwendung zur Behandlung oder Prophylaxe systemischer oder topischer mikrobieller Infektionen in einem menschlichen oder tierischen Körper.

11. Pharmazeutische Zusammensetzung, die wenigstens eine Verbindung, wie in einem der Ansprüche 1 bis 7 beansprucht, in Gemeinschaft mit einem pharmazeutisch annehmbaren Hilfsstoff, Verdünnungsmittel und/oder Trägerstoff umfaßt.

12. Verbindung der Formel (IA) 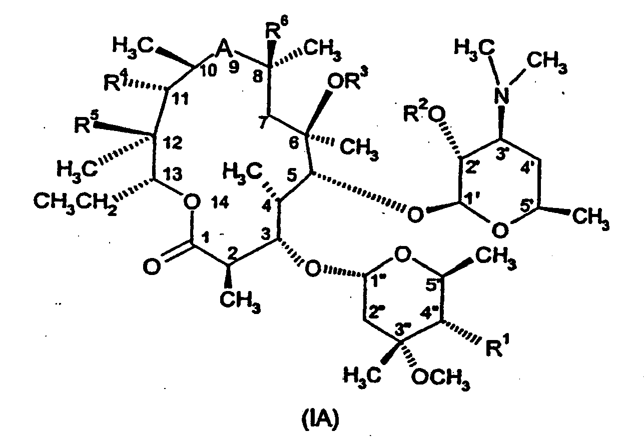 worin
A ein bivalenter Rest ist, ausgewählt aus -C(O)-, -C(O)NH-, -NHC(O)-, -N(R⁷)-CH₂-, -CH₂-N(R⁷)-, -CH(NR⁸R⁹)- und -C(=NR¹⁰)-;
R¹ ist -OC(O)N(R¹¹)(CH_{2)d}XR¹²;
R² ist Wasserstoff oder eine Hydroxyl-Schutzgruppe;
R³ ist Wasserstoff, C₁₋₄-Alkyl oder C₃₋₆-Alkenyl, das gegebenenfalls durch ein 9-bis 10-gliedriges, anneliertes bicyclisches Heteroaryl substituiert ist;
R⁴ ist Hydroxy, C₃₋₆-Alkenyloxy, das gegebenenfalls durch ein 9- bis 10-gliedriges, anneliertes bicyclisches Heteroaryl substituiert ist, oder C₁₋₆-Alkoxy, das gegebenenfalls durch C₁₋₆-Alkoxy oder -O(CH₂)ₑNR⁷R¹³ substituiert ist;
R⁵ ist Hydroxy, oder
R⁴ und R⁵ bilden zusammen mit den dazwischenliegenden Atomen eine cyclische Gruppe, die die folgende Struktur hat: 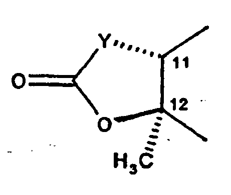 worin Y eine bivalenter Rest ist, ausgewählt aus -CH₂-, -CH(CN)-, -O-, -N(R¹⁴)- und -CH(SR¹⁴)-;
R⁶ ist Wasserstoff oder Fluor;
R⁷ ist Wasserstoff oder C₁₋₆-Alkyl;
R⁸ und R⁹ sind jeweils unabhängig Wasserstoff, C₁₋₆-Alkyl, -C(=NR¹⁰)NR¹⁵R¹⁶ oder -C(O)R¹⁵, oder
R⁸ und R⁹ bilden zusammen =CH(CR¹⁵R¹⁶)_{f}-Aryl, =CH(CR¹⁵R¹⁶)_{f}-Heterocyclyl, =CR¹⁵R¹⁶ oder =C(R¹⁵)C(O)OR¹⁵, worin die Alkyl-, Aryl- und Heterocyclyl-Gruppen gegebenenfalls durch bis zu drei Gruppen substituiert sind, die unabhängig aus R¹⁷ ausgewählt sind;
R¹⁰ ist -OR¹⁸, C₁₋₆-Alkyl, -(CH₂)_{g}-Aryl, -(CH₂)_{g}-Heterocyclyl oder -(CH₂)ₕO(CH₂)ᵢOR⁷, worin jede R¹⁰-Gruppe gegebenenfalls durch bis zu drei Gruppen, unabhängig aus R¹⁷ ausgewählt, substituiert sind;
R¹¹ ist Wasserstoff oder C₁₋₆-Alkyl;
R¹² ist eine heterocyclische Gruppe, die die folgende Struktur hat: 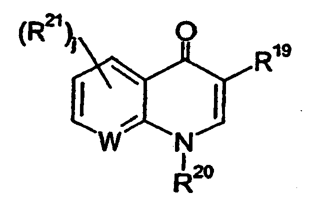 oder 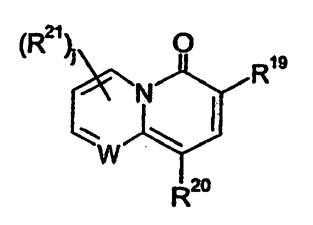 R¹³ ist Wasserstoff oder C₁₋₆-Alkyl;
R¹⁴ ist Wasserstoff oder C₁₋₄-Alkyl, das durch eine Gruppe, ausgewählt aus gegebenenfalls substituiertem Phenyl, einem gegebenenfalls substituierten 5- oder 6-gliedrigen Heteroaryl und einem gegebenenfalls substituierten 9- bis 10-gliedrigen annelierten bicyclischen Heteroaryl, substituiert ist;
R¹⁵ und R¹⁶ sind jeweils unabhängig Wasserstoff oder C₁₋₆-Alkyl;
R¹⁷ ist Halogen, Cyano, Nitro, Trifluormethyl, Azido, -C(O)R²², -C(O)OR²², -OC(O)R²², -OC(O)OR²², -NR²²C(O)R²⁴, -C(O)NR²³R²⁴, -NR²³R²⁴, Hydroxy, C₁₋₆-Alkyl, -S(O)ₖ-C₁₋₆-Alkyl, C₁₋₆-Alkoxy, -(CH₂)ₘ-Aryl oder -(CH₂)ₘ-Heteroaryl, worin die Alkoxy-Gruppe durch bis zu drei Gruppen substituiert ist, unabhängig ausgewählt aus -NR¹⁵R¹⁶, Halogen und -OR¹⁵ und die Aryl- und Heteroaryl-Gruppen sind gegebenenfalls durch bis zu fünf Gruppen, unabhängig ausgewählt aus Halogen, Cyano, Nitro, Trifluormethyl, Azido, -C(O)R²⁵, -C(O)OR²⁵, -OC(O)OR²⁵, -NR²⁶C(O)R²⁷, -C(O)NR²⁶R²⁷, -NR²⁶R²⁷, Hydroxy, C₁₋₆-Alkyl und C₁₋₆-Alkoxy, substituiert;
R¹⁸ ist Wasserstoff, C₁₋₆-Alkyl, C₃₋₇-Cycloalkyl, C₃₋₆-Alkenyl oder eine 5- oder 6-gliedrige heterocyclische Gruppe, worin die Alkyl-, Cycloalkyl-, Alkenyl- und heterocyclischen Gruppen gegebenenfalls durch bis zu drei Substituenten substituiert sind, die unabhängig aus einer gegebenenfalls substituierten 5- oder 6-gliedrigen heterocyclischen Gruppe, gegebenenfalls einem substituierten 5- oder 6-gliedrigen Heteroaryl, -OR²⁸, -S(O)NR²⁸, -NR²⁸R²⁹, -CONR²⁸R²⁹, Halogen und Cyano ausgewählt sind;
R¹⁹ ist Wasserstoff, -C(O)OR³⁰, -C(O)NHR³⁰, -C(O)CH₂NO₂;
R²⁰ ist Wasserstoff, C₁₋₄-Alkyl, gegebenenfalls durch Hydroxy oder C₁₋₄-Alkoxy substituiert, C₃₋₇-Cycloalkyl, oder gegebenenfalls durch Phenyl oder Benzyl substituiert;
R²¹ ist Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂;
R²² ist Wasserstoff, C₁₋₁₀-Alkyl, -(CH₂)ₚ-Aryl oder -(CH₂)ₚ-Heteroaryl;
R²³ und R²⁴ sind jeweils unabhängig Wasserstoff, -OR¹⁵, C₁₋₆-Alkyl, -(CH₂)_{q}-Aryl oder -(CH₂)_{q}-Heterocyclyl;
R²⁵ ist Wasserstoff, C₁₋₁₀-Alkyl, -(CH₂)ᵣ-Aryl oder -(CH₂)ᵣ-Heteroaryl;
R²⁶ und R²⁷ sind jeweils unabhängig Wasserstoff, -OR¹⁵, C₁₋₆-Alkyl, -(CH₂)ₛ-Aryl oder -(CH₂)ₛ₋Heterocyclyl;
R²⁸ und R²⁹ sind jeweils unabhängig Wasserstoff, C₁₋₄-Alkyl oder C₁₋₄-Alkoxy-C₁₋₄-Alkyl;
R³⁰ ist Wasserstoff, C₁₋₆-Alkyl, das gegebenenfalls durch bis zu drei Gruppen substituiert ist, unabhängig ausgewählt aus Halogen, C₁₋₄-Alkoxy, -OC(O)C₁₋₆-Alkyl und -OC(O)OC₁₋₆-Alkyl;
R³¹ ist Wasserstoff, C₁₋₄-Alkyl, C₃₋₇-Cycloalkyl, das gegebenenfalls durch Phenyl oder Benzyl substituiert ist, Acetyl oder Benzoyl;
R³² ist Wasserstoff oder R²¹, oder R³² und R²⁰ sind verbunden, um den bivalenten Rest -O(CH₂)₂- oder -(CH₂)t- zu bilden;
X ist -U(CH₂)ᵥB-, -U(CH₂)ᵥ- oder eine Gruppe, ausgewählt aus: 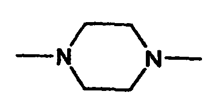 und 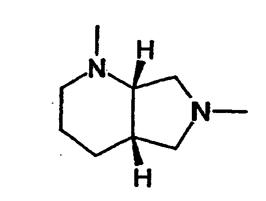 U und B sind unabhängig ein zweiwertiger Rest, ausgewählt aus -N(R³¹)-, -O-, -S(O)_{z}-, -N(R³¹)C(O)-, -C(O)N(R³¹)- und -N[C(O)R³¹]-;
W ist -C(R³²)- oder ein Stickstoffatom;
d ist eine ganze Zahl von 2 bis 5;
e ist eine ganze Zahl von 2 bis 4;
f, g, h, m, p, q, r und s sind jeweils unabhängig ganze Zahlen von 0 bis 4;
i ist eine ganze Zahl von 1 bis 6;
j, k, n und z sind jeweils unabhängig ganze Zahlen von 0 bis 2;
t ist 2 oder 3;
v ist eine ganze Zahl von 1 bis 8;
oder eines pharmazeutisch annehmbaren Derivates davon.

## Revendications

1. Composé de formule (I) 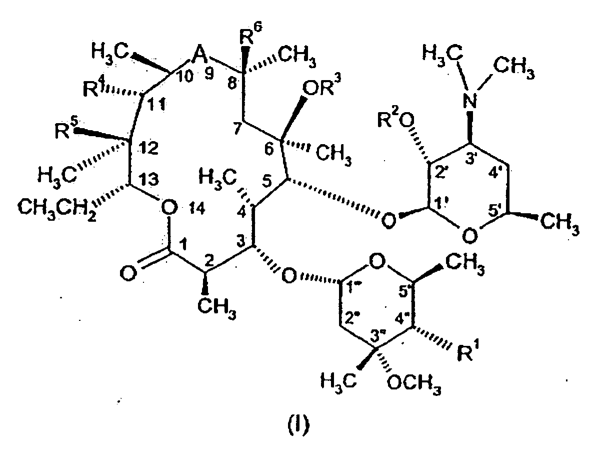 dans laquelle
- A représente un radical bivalent choisi parmi les groupes -C(O)-, -C(O)NH-,-NHC(O)-, -N(R⁷)-CH₂-, -CH₂-N(R⁷)-, -CH(NR⁸R⁹)- et -C(=NR¹⁰)-;
- R¹ représente un groupe -OC(O)N(R¹¹)(CH₂)_{d}XR¹² ;
- R² représente l'hydrogène ou un groupe protecteur d'hydroxyle ;
- R³ représente l'hydrogène, un groupe alkyle C₁₋₄ ou alkényle C₃₋₆ éventuellement substitué par un groupe hétéroaryle bicyclique fusionné de 9 à 10 atomes ;
- R⁴ représente un groupe hydroxy, alkényl(C₃₋₆)oxy éventuellement substitué par un groupe hétéroaryle bicyclique fusionné de 9 à 10 atomes, ou un groupe alcoxy éventuellement substitué par un groupe alcoxy C₁₋₆, ou un groupe -O(CH₂)ₑNR⁷R¹³;
- R⁵ représente un groupe hydroxy ou bien
- R⁴ et R⁵ pris en association avec les atomes intervenants forment un groupe cyclique ayant la structure suivante : 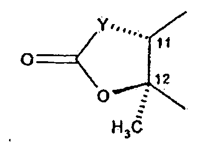 dans laquelle Y représente un radical bivalent choisi parmi -CH₂-, -CH(CN)-, -O-, -N(R¹⁴)- et -CH(SR¹⁴)-;
- R⁶ représente l'hydrogène ou le fluor ;
- R⁷ représente l'hydrogène ou un groupe alkyle C₁₋₆;
- R⁸ et R⁹ représentent chacun indépendamment l'hydrogène, un groupe alkyle C₁₋₆, -C(=NR¹⁰)NR¹⁵R¹⁶ ou -C(O)R¹⁵, ou bien
- R⁸ et R⁹ en association, forment un groupe =CH(CR¹⁵R¹⁶)_{f}aryle, =CH(CR¹⁵R¹⁶)_{f}hétérocyclyle, =CR¹⁵R¹⁶ ou =C(R¹⁵)C(O)OR¹⁵ où les groupes alkyle, aryle et hétérocyclyle sont éventuellement substitués par jusqu'à trois groupes choisis indépendamment parmi les groupes R¹⁷ ;
- R¹⁰ représente un groupe -OR¹⁸, alkyle C ₁₋₆, -(CH₂)_{g}aryle, -(CH₂)_{g}-hétérocyclyle ou -(CH₂)ₕO(CH₂)ᵢOR⁷ où chaque groupe R¹⁰ est éventuellement substitué par jusqu'à trois groupes choisis indépendamment parmi les groupes R¹⁷ ;
- R¹¹ représente l'hydrogène ou un groupe alkyle C₁₋₆,
- R¹² représente un groupe hétérocyclique ayant la structure suivante : 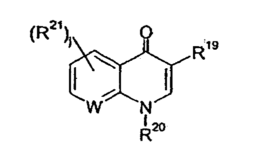 ou 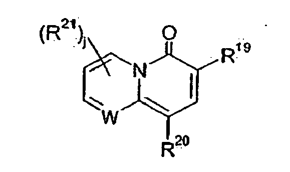
- R¹³ représente l'hydrogène ou un groupe alkyle C₁₋₆ ;
- R¹⁴ représente l'hydrogène ou un groupe alkyle C₁₋₄ éventuellement substitué par un groupe choisi parmi les groupes phényle éventuellement substitué, hétéroaryle à 5 ou 6 atomes, éventuellement substitué et hétéroaryle bicyclique fusionné de 9 à 10 atomes, éventuellement substitué ;
- R¹⁵ et R¹⁶ représentent chacun indépendamment, l'hydrogène ou un groupe alkyle C₁₋₆ :
- R¹⁷ représente un halogène, un groupe cyano, nitro, trifluorométhyle, azido, -C(O)R²², -C(O)OR²², -OC(O)R²², -OC(O)OR²², -NR²³C(O)R²⁴, -C(O)NR²³R²⁴, -NR²³R²⁴, hydroxy, alkyle C₁₋₆, -S(O)ₖalkyle C₁₋₆, alcoxy C₁₋₆, -(CH₂)ₘaryle, ou -(CH₂)ₘhétéroaryle, où le groupe alcoxy est éventuellement substitué par jusqu'à trois groupes choisis indépendamment parmi les groupes -NR¹⁵R¹⁶, halogène et -OR¹⁵, et où les groupes aryle et hétéroaryle sont éventuellement substitués par jusqu'à cinq groupes choisis indépendamment parmi les groupes halogène, cyano, nitro, trifluorométhyle, azido, -C(O)R²⁵, -C(O)OR²⁵, -OC(O)OR²⁵, -NR²⁶C(O)R²⁷, -C(O)NR²⁶R²⁷, -NR²⁶R²⁷, hydroxy, alkyle C₁₋₆ et alcoxy C₁₋₆ ;
- R¹⁸ représente l'hydrogène, un groupe alkyle C₁₋₆, cycloalkyle C₃₋₇, alkényle C₃₋₆ ou un groupe hétérocyclique à 5 ou 6 atomes, où les groupes alkyle, cycloalkyle, alkényle et hétérocyclique sont éventuellement substitués par jusqu'à trois substituants choisis indépendamment parmi un groupe hétérocyclique à 5 ou 6 atomes éventuellement substitué, un groupe hétéroaryle à 5 ou 6 atomes éventuellement substitué, un groupe -OR²⁸, -S(O)ₙR²⁸, -NR²⁸R²⁹, -CONR²⁸R²⁹, halogène et cyano ;
- R¹⁹ représente l'hydrogène, un groupe -C(O)OR³⁰, -C(O)NHR³⁰, -C(O)CH₂NO₂ ou -C(O)CH₂SO₂R⁷ ;
- R²⁰ représente l'hydrogène, un groupe alkyle C₁₋₄ éventuellement substitué par un groupe hydroxy ou alcoxy C₁₋₄, cycloalkyle C ₃₋₇ ou phényle ou benzyle éventuellement substitué ;
- R²¹ représente un halogène, un groupe alkyle C₁₋₄, thioalkyle C₁₋₄, alcoxy C₁₋₄, -NH₂, -NH-alkyle C₁₋₄ ou -N(alkyle C₁₋₄)₂ ;
- R²² représente l'hydrogène, un groupe alkyle C₁₋₁₀, -(CH₂)ₚaryle ou -(CH₂)ₚhétéroaryle ;
- R²³ et R²⁴ représentent chacun indépendamment l'hydrogène, un groupe -OR¹⁵, alkyle C₁₋₆, -(CH₂)_{q}aryle ou -(CH₂)_{q}hétérocyclyle ;
- R²⁵ représente l'hydrogène, un groupe alkyle C₁₋₁₀, -(CH₂)ᵣaryle ou -(CH₂)ᵣhétéroaryle ;
- R²⁶ et R²⁷ représentent chacun indépendamment l'hydrogène, un groupe -OR¹⁵, alkyle C₁₋₆, -(CH₂)ₛaryle ou -(CH₂)ₛhétérocyclyle ;
- R²⁸ et R²⁹ représentent chacun indépendamment l'hydrogène, un groupe alkyle C₁₋₄ ou alcoxy(C₁₋₄)alkyle C₁₋₄;
- R³⁰ représente
l'hydrogène
un groupe alkyle C₁₋₆ éventuellement substitué par jusqu'à trois groupes choisis indépendamment parmi les halogènes, les groupes cyano, alcoxy C₁₋₄ éventuellement substitués par un phényle ou des groupes alcoxy C₁₋₄, -C(O)alkyle C₁₋₆, -C(O)O-alkyle C₁₋₆, -OC(O)-alkyle C₁₋₆,
- OC(O)O-alkyle C₁₋₆, -C(O)NR³³R³⁴, -NR³³R³⁴ et phényle éventuellement substitué par un groupe nitro ou -C(O)O-alkyle C₁₋₆,
un groupe -(CH₂)_{w}cycloalkyle C₃₋₇,
un groupe -(CH₂)_{w}hétérocyclyle,
un groupe -(CH₂)_{w}hétéroaryle,
un groupe -(CH₂)_{w}aryle,
un groupe alkényle C₃₋₆, ou
un groupe alkynyle C₃₋₆ ;
- R³¹ représente l'hydrogène, un groupe alkyle C₁₋₄, cycloalkyle C₃₋₇, phényle ou benzyle éventuellement substitué, acétyle ou benzoyle ;
- R³² représente l'hydrogène ou R²¹, ou bien R³² et R²⁰ sont liés pour former le radical bivalent -O(CH₂)₂- ou -(CH₂)₁-;
- R³³ et R³⁴ représentent chacun indépendamment l'hydrogène ou un groupe alkyle C₁₋₆ éventuellement substitué par un groupe phényle ou -C(O)O-alkyle C₁₋₆, ou bien
- R³³ et R³⁴ associés à l'atome d'azote auquel ils sont liés, forment un groupe hé térocyclique à 5 ou 6 atomes contenant éventuellement un hétéroatome additionnel choisi parmi l'oxygène, l'azote ou le soufre ;
- X représente un groupe -U(CH₂)ᵥB-, -U(CH₂)ᵥ-, ou un groupe choisi parmi : 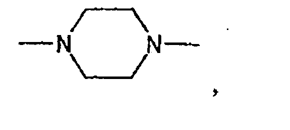 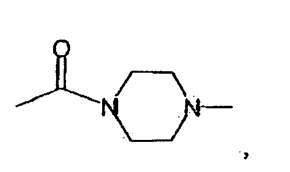 et 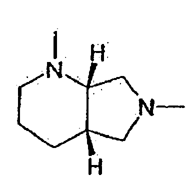
- U et B représentent indépendamment un radical divalent choisi parmi -N(R³¹)-, -O-, -S(O)₂-, -N(R³¹)C(O)-, -C(O)N(R³¹)- et -N[C(O)R³¹]- ;
- W représente un groupe -C(R³²)- ou un atome d'azote ;
- d est un nombre entier de 2 à 5 ;
- e est un nombre entier de 2 à 4 ;
- f, g, h, m, p, q, r, s et w sont chacun indépendamment des nombres entiers de 0 à 4 ;
- i est un nombre entier de 1 à 6 ;
- j, k, n et z sont chacun indépendamment des nombres entiers de 0 à 2 ;
- t est égal à 2 ou à 3 ;
- v est un nombre entier de 1 à 8 ;
ou un dérivé pharmaceutiquement acceptable de celui-ci.

2. Composé selon la revendication 1, dans lequel A représente -C(O)- ou -N(R⁷)-CH₂-.

3. Composé selon la revendication 1 ou la revendication 2 dans lequel X représente -U(CH₂)ᵥB ou -U(CH₂)ᵥ.

4. Composé selon l'une quelconque des revendications précédentes, dans lequel d est égal à 2 ou à 3.

5. Composé selon l'une quelconque des revendications précédentes, dans lequel R¹² représente un groupe hétérocyclique de formule suivante : 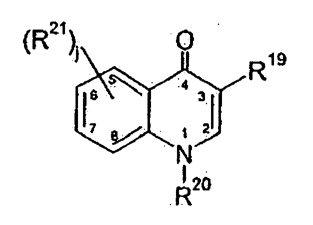 dans laquelle l'hétérocyclique est lié en position 6 ou en position 7, et j, R¹⁹, R²⁰ et R²¹ sont tels que définis dans la revendication 1.

6. Composé selon la revendication 1, tel que défini dans l'un quelconque des Exemples 1 à 20, ou dérivé pharmaceutiquement acceptable de celui-ci.

7. Composé choisi parmi les suivants :
- 4"-*O*-3-[2-(3-Carboxy-7-chloro-1-cyclopropyl-4-oxo-1,4-dihydroquinoléin-6-yloxy)éthoxy]propylcarbamoyloxy}azithromycine ;
- 4"-*O*-{3-[2-(3-Carboxy-1-cyclopropyl-4-oxo-1,4-dihydroquinoléin-6-yloxy)éthoxy] propylcarbamoyloxy}azithromycine ;
- 4"-*O*-{2-[3-(3-Carboxy-1-éthyl-1-oxo-1,4-dihydroquinoléin-6-yl)propyloxy]éthylcarbamoyl}-6-*O*-méthyl-érythromycine A ;
- 4"-*O*-{3-[2-(3-Carboxy-1-cyclopropyl-4-oxo-1,4-dihydroquinoléin-6-ylamino)éthoxy]propylcarbamoyl}azithromycine ;
- 4"-*O*-{3-[2-(3-Carboxy-1-cyclopropyl-4-oxo-1,4-dihydroquinoléin-6-yloxy)éthoxy]propylcarbamoyl}-11-*O*-méthylazithromycine ; et
- 4"-*O*-{3-[2-(3-Carboxy-1-cyclopropyl-4-oxo-1,4-dihydroquinoléin-6-ylamino)éthoxy]propylcarbamoyl}-11-*O*-méthylazithromycine ;
ou un dérivé pharmaceutiquement acceptable de ceux-ci.

8. Procédé de préparation d'un composé selon la revendication 1, qui comprend :
(a) la réaction d'un composé de formule (II) 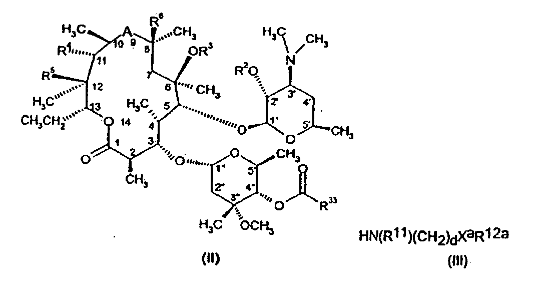 avec une amine appropriée (III) où R³³ est un groupe activant, et X^{a} et R^{12a} représentent X et R¹² tels que définis dans la revendication 1 ou des groupes convertibles en X et R¹².
(b) la réaction d'un composé de formule (IV) 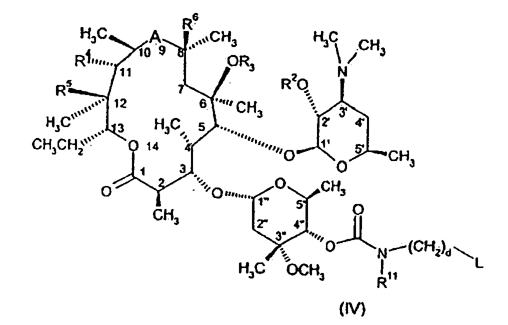 avec un composé de formule X^{a}R^{12a} (V) dans laquelle R^{12a} représente R¹²_{.} tel que défini dans la revendication 1 ou bien est un groupe convertible en R¹², et X^{a} représente -U(CH₂)ᵥ- ou -U(CH₂)ᵥB-, ou un groupe convertible en -U(CH₂)ᵥ- ou en -U(CH₂)ᵥB-, où U est un groupe choisi parmi -N(R³¹)- et -S-, et L est un groupe partant approprié, pour produire un composé de formule (I) dans laquelle U est un groupe choisi parmi -N(R³¹)- et -S- ; ou
(c) la conversion d'un composé de formule (I) en un autre composé de formule (I) ;
et ensuite, si besoin est, la soumission du composé obtenu à une ou plusieurs des opérations suivantes :
(i) déplacement du groupe protecteur R²,
(ii) conversion de X^{a}R^{12a} en XR¹², et
(iii) conversion du composé de formule (II) obtenu en un dérivé pharmaceutiquement acceptable de celui-ci.

9. Composé selon l'une quelconque des revendications 1 à 7, en vue de l'utilisation en thérapie.

10. Utilisation d'un composé selon l'une quelconque des revendications 1 à 7 dans la fabrication d'un médicament en vue de l'utilisation dans le traitement ou la prophylaxie d'infections microbiennes systémiques ou topiques dans un corps humain ou animal.

11. Composition pharmaceutique comprenant au moins un composé selon l'une quelconque des revendications 1 à 7, en association avec un excipient, diluant et/ou véhicule pharmaceutiquement acceptables.

12. Composé de formule (IA) 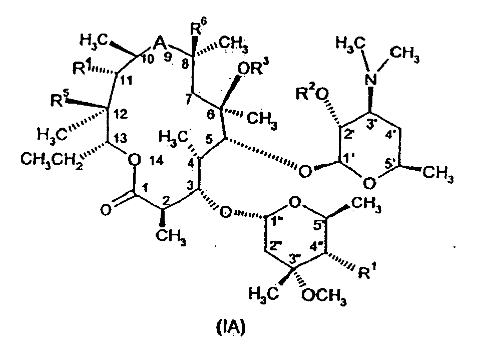 dans laquelle
- A représente un radical bivalent choisi parmi les groupes -C(O)-, -C(O)NH-, -NHC(O)-, -N(R⁷)-CH₂-, -CH₂-N(R⁷)-, -CH(NR⁸R⁹)- et -C(=NR¹⁰)- ;
- R¹ représente un groupe -OC(O)N(R¹¹)(CH₂)_{d}XR¹² ;
- R² représente l'hydrogène ou un groupe protecteur d'hydroxyle ;
- R³ représente l'hydrogène, un groupe alkyle C₁₋₄ ou alkényle C₃₋₆, éventuellement substitué par un groupe hétéroaryle bicyclique fusionné de 9 à 10 atomes ;
- R⁴ représente un groupe hydroxy, alkényl(C₃₋₆)oxy éventuellement substitué par un groupe hétéroaryle bicyclique fusionné de 9 à 10 atomes, ou un groupe alcoxy C₁₋₆ éventuellement substitué par un groupe alcoxy C₁₋₆, ou un groupe -O(CH₂)ₑNR⁷R¹³;
- R⁵ représente un groupe hydroxy ou bien
- R⁴ et R⁵ pris en association avec les atomes intervenants forment un groupe cyclique ayant la structure suivante : 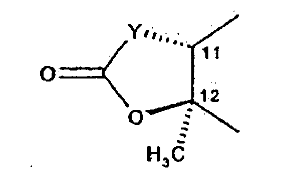 dans laquelle Y représente un radical bivalent choisi parmi -CH₂-, -CH(CN)-, -O-, -N(R¹⁴)- et -CH(SR¹⁴)-;
- R⁶ représente l'hydrogène ou le fluor ;
- R⁷ représente l'hydrogène ou un groupe alkyle C₁₋₆;
- R⁸ et R⁹ représentent chacun indépendamment l'hydrogène, un groupe alkyle C₁₋₆, -C(=NR¹⁰)NR¹⁵R¹⁶ ou -C(O)R¹⁵, ou bien
- R⁸ et R⁹ en association, forment un groupe =CH(CR¹⁵R¹⁶)_{f}aryle, =CH(CR¹⁵R¹⁶)_{f}hétérocyclyle, =CR¹⁵R¹⁶ ou =C(R¹⁵)C(O)OR¹⁵ où les groupes alkyle, aryle et hétérocyclyle sont éventuellement substitués par jusqu'à trois groupes choisis indépendamment parmi les groupes R¹⁷;
- R¹⁰ représente un groupe -OR¹⁸, alkyle C₁₋₆, -(CH₂)_{g}aryle, -(CH₂)_{g}-hétérocyclyle ou -(CH₂)ₕO(CH₂)ᵢOR⁷ où chaque groupe R¹⁰ est éventuellement substitué par jusqu'à trois groupes choisis indépendamment parmi les groupes R¹⁷;
- R¹¹ représente l'hydrogène ou un groupe alkyle C₁₋₆;
- R¹² représente un groupe hétérocyclique ayant la structure suivante: 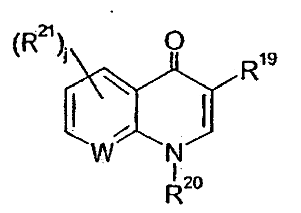 ou 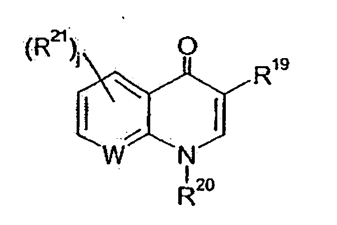
- R¹³ représente l'hydrogène ou un groupe alkyle C₁₋₆;
- R¹⁴ représente l'hydrogène ou un groupe alkyle C₁₋₄ substitué par un groupe choisi parmi les groupes phényle éventuellement substitué, hétéroaryle à 5 ou 6 atomes éventuellement substitué et hétéroaryle bicyclique fusionné de 9 à 10 atomes, éventuellement substitué;
- R¹⁵ et R¹⁶ représentent chacun indépendamment, l'hydrogène ou un groupe alkyle C₁₋₆:
- R¹⁷ représente un halogène, un groupe cyano, nitro, trifluorométhyle, azido, -C(O)R²², -C(O)OR²², -OC(O)R²², -OC(O)OR²², -NR²³C(O)R²⁴, -C(O)NR²³R²⁴, -NR²³R²⁴, hydroxy, alkyle C₁₋₆, -S(O)ₖalkyle C₁₋₆, alcoxy C₁₋₆, -(CH₂)ₘaryle, ou -(CH₂)ₘhétéroaryle, où le groupe alcoxy est éventuellement substitué par jusqu'à trois groupes choisis indépendamment parmi les groupes -NR¹⁵R¹⁶, halogène et -OR¹⁵, et où les groupes aryle et hétéroaryle sont éventuellement substitués par jusqu'à cinq groupes choisis indépendamment parmi les groupes halogène, cyano, nitro, trifluorométhyle, azido, -C(O)R²⁵, -C(O)OR²⁵, -OC(O)OR²⁵, -NR²⁶C(O)R²⁷, -C(O)NR²⁶R²⁷, -NR²⁶R²⁷, hydroxy, alkyle C₁₋₆ et alcoxy C₁₋₆;
- R¹⁸ représente l'hydrogène, un groupe alkyle C₁₋₆, cycloalkyle C₃₋₇, alkényle C₃₋₆ ou un groupe hétérocyclique à 5 ou 6 atomes, où les groupes alkyle, cycloalkyle, alkényle et hétérocyclique sont éventuellement substitués par jusqu'à trois substituants choisis indépendamment parmi un groupe hétérocyclique à 5 ou 6 atomes éventuellement substitué, un groupe hétéroaryle à 5 ou 6 atomes éventuellement substitué, un groupe -OR²⁸, -S(O)ₙR²⁸, -NR²⁸R²⁹, -CONR²⁸R²⁹, halogène et cyano ;
- R¹⁹ représente l'h ydrogène, un groupe - C(O)OR³⁰, -C(O)NHR³⁰ ou -C(O)CH₂NO₂;
- R²⁰représente l'hydrogène, un groupe alkyle C₁₋₄ éventuellement substitué par un hydroxy ou un alcoxy C₁₋₄, cycloalkyle C₃₋₇ ou phényle ou benzyle éventuellement substitué;
- R²¹ représente un halogène, un groupe alkyle C₁₋₄, thioalkyle C₁₋₄, alcoxy C₁₋₄, -NH₂, -NH-alkyle C₁₋₄ ou -N(alkyle C₁₋₄)₂;
- R²² représente l'hydrogène, un groupe alkyle C₁₋₁₀, -(CH₂)paryle ou -(CH₂)ₚhétéroaryle;
- R²³ et R²⁴ représentent chacun indépendamment l'hydrogène, un groupe -OR¹⁵, alkyle C₁₋₆, -(CH₂)_{q}aryle ou -(CH₂)_{q}hétérocyclyle;
- R²⁵ représente l'hydrogène, un groupe alkyle C₁₋₁₀, -(CH₂)ᵣaryle ou -(CH₂)ᵣhétéroaryle;
- R²⁶ et R²⁷ représentent chacun indépendamment l'hydrogène, un groupe -OR¹⁵, alkyle C₁₋₆, -(CH₂)ₛaryle ou -(CH₂)ₛhétérocyclyle;
- R²⁸ et R²⁹ représentent chacun indépendamment l'hydrogène, un groupe alkyle C₁₋₄ ou alcoxy(C₁₋₄)alkyle C₁₋₄;
- R³⁰ représente l'hydrogène ou un groupe alkyle C₁₋₆ éventuellement substitué par jusqu'à trois groupes choisis indépendamment parmi les halogènes, les groupes alcoxy C₁₋₄, -OC(O)alkyle C₁₋₆ et -OC(O)O-alkyle C₁₋₆;
- R³¹ représente l'hydrogène, un groupe alkyle C₁₋₄, cycloalkyle C₃₋₇, phényle ou benzyle éventuellement substitué, acétyle ou benzoyle;
- R³² représente l'hydrogène ou R²¹, ou bien R³² et R²⁰ sont liés pour former le radical bivalent -O(CH₂)₂- ou -(CH₂)ₜ-;
- X représente un groupe -U(CH₂)ᵥB-, -U(CH₂)ᵥ-, ou un groupe choisi parmi : 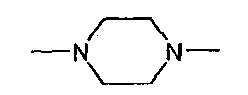
et 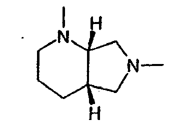
- U et B représentent indépendamment un radical divalent choisi parmi -N(R³¹)-, -O-, -S(O)₂-, -N(R³¹)C(O)-, -C(O)N(R³¹) et -N[C(O)R³¹]-;
- W représente un groupe -C(R³²)- ou un atome d' azote ;
- d est un nombre entier de 2 à 5;
- e est un nombre entier de 2 à 4;
- f, g, h, m, p, q, r et s sont chacun indépendamment des nombres entiers de 0 à 4 ;
- i est un nombre entier de 1 à 6;
- j, k, n et z sont chacun indépendamment des nombres entiers de 0 à 2;
- t est égal à 2 ou à 3 ;
- v est un nombre entier de 2 à 8;
ou un dérivé pharmaceutiquement acceptable de celui-ci.
